(19) Europäisches Patentamt
European Patent Office
Office européen des brevets

(11) **EP 2 736 483 B1**

(12) **EUROPÄISCHE PATENTSCHRIFT**

(45) Veröffentlichungstag und Bekanntmachung des Hinweises auf die Patenterteilung:
**13.04.2016 Patentblatt 2016/15**

(21) Anmeldenummer: **12742848.0**

(22) Anmeldetag: **24.07.2012**

(51) Int Cl.:
*A61Q 5/02* (2006.01)        *A61Q 5/12* (2006.01)
*A61K 8/898* (2006.01)       *A61K 8/04* (2006.01)

(86) Internationale Anmeldenummer:
**PCT/EP2012/064461**

(87) Internationale Veröffentlichungsnummer:
**WO 2013/014140 (31.01.2013 Gazette 2013/05)**

(54) **KOSMETISCHE ZUSAMMENSETZUNGEN**

COSMETIC COMPOSITIONS

COMPOSITIONS COSMETIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.07.2011 DE 102011079911**

(43) Veröffentlichungstag der Anmeldung:
**04.06.2014 Patentblatt 2014/23**

(73) Patentinhaber: **Wacker Chemie AG**
**81737 München (DE)**

(72) Erfinder:
• **BEER, Gerhard**
**84489 Burghausen (DE)**

• **SCHWARZWÄLDER, Claudius**
**84489 Burghausen (DE)**

(74) Vertreter: **Deffner-Lehner, Maria et al**
**Wacker Chemie AG**
**Zentralbereich Patente, Marken und Lizenzen**
**Hanns-Seidel-Platz 4**
**81737 München (DE)**

(56) Entgegenhaltungen:
**EP-A1- 1 921 203        EP-A2- 0 992 528**
**WO-A1-2006/119916**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft die Verwendung kosmetischer Zusammensetzungen enthaltend - in einem kosmetisch akzeptablen Medium - mindestens ein Konditionierungsmittel und mindestens ein Organopolysiloxan.

Organopolysiloxane werden in kosmetischen Zusammensetzungen, beispielsweise bei Haarpflegeprodukten, verwendet aufgrund ihrer konditionierenden Eigenschaften wie Verbesserung von Weichheit und Glätte, Reduzierung von Kämmkräften, Glanzeigenschaften, Verbesserung von Farbeindrücken, Farbschutzeigenschaften, Verringerung der elektrostatischen Aufladungen, Schutzeigenschaften bei thermischer Belastung des Haars oder Hydrophobisierung.

Eine Übersicht über ausgewählte Organopolysiloxane für Pflege keratinischer Materialien wie Haaren findet sich in M.D. Berthiaume, Society of Cosmetic Chemists (Hrsg.), Monograph, Silicones in Hair Care, 1997 und J. Sejpka, Silicone in Haarpflegeprodukten, in: SÖFW-Journal, 118. Jahrgang, Nr. 17, 1992, S. 1065-1070,.

Haare sind im Alltag einer Vielzahl von äußeren Einflüssen ausgesetzt, die zur Schädigung der Haaroberfläche führen, und damit die kosmetischen Eigenschaften wie Glätte, Weichheit, Glanz und andere Parameter im Vergleich zu ungeschädigtem Haar verschlechtern. Die Schädigung der Haaroberfläche kann beispielsweise durch chemische oder mechanische Behandlung verursacht sein, durch UV-Strahlung oder durch Hitze. Einhergehend mit der Oberflächenschädigung von Haar ist die Zerstörung und teilweise Entfernung der die Cuticula bedeckenden Lipidschicht, die ursächlich für die stark hydrophobe Eigenschaft von ungeschädigtem, natürlichem Haar ist (R.A. Lodge, B. Bhusan, Wetting Properties of Human Hair by Means of Dynamic Contact Angle Measurement, Journal of Applied Polmyer Science, Vol. 102, 5255-5265, 2006, Wiley). Geschädigtes Haar ist im Vergleich zu ungeschädigtem Haar deutlich hydrophiler, da nach Zerstörung der oberflächlichen Lipidschicht eine hydrophile, aminosäurebasierte Proteinmatrix als Haaroberfläche wirkt.

In US 7,223,385 B2, US 7,485,289 B2, US 7,220,408 B2 und US 7,504,094 B2 werden kosmetische Zusammensetzungen zur Behandlung von Haaren beschrieben, die spezielle Aminosilicone der Struktur (I) oder (II) enthalten sowie ein Konditionierungsmittel oder einen Verdicker. Bei den Aminosiliconen der Struktur (I) oder (II) handelt es sich um endständige Alkoxy/Hydroxygruppen aufweisende Dimethylpolysiloxane mit Aminoethylaminopropyl-Alkoxy-Siloxaneinheiten oder Aminoethylaminopropyl-Methyl-Siloxaneinheiten, die unvernetzt sind.

Gemäß US 2008/0064813 A1 werden wässrige Dispersionen von vorvernetzten Organopolysiloxanen ohne Mitverwendung von metallhaltigen Katalysatoren erhalten durch Umsetzung von Alkoxy- oder Hydroxygruppen aufweisende Organopolysiloxane mit reaktiven Alkoxysilanen, die eine die Reaktivität der Alkoxygruppe steigernde Gruppe, wie eine Methylenaminogruppe, aufweisen. Nach Auftragen der Dispersionen auf Substraten und Verdampfen des Wassers werden elastomere Filme erhalten.

Aus EP 1921 203 A1 ist ein Verfahren zur Behandlung von Füllfasern mit wässrigen Dispersionen von Organopolysiloxanen ohne Mitverwendung von Metall-haltigen Katalysatoren bekannt.

In EP 992 528 A2 ist die Verwendung einer Siliconemulsion, die durch Umsetzung einer wässrigen Emulsion eines hydroxyendständigen Polyorganosiloxans mit einem aminofunktionellen Silan in Haarbehandlungszusammensetzungen beschrieben.

Aufgabe der Erfindung ist, eine Verbesserung kosmetischer Parameter für geschädigtes Haar, wie Weichheit, Glätte, Kämmkräfte, Glanz- und Farbeigenschaften, Verringerung der elektrostatischen Aufladungen und Schutzeigenschaften bei thermischer Belastung zu erzielen. Insbesondere bestand die Aufgabe, die Hydrophobie der Haaroberfläche zu erhöhen.

Gegenstand der Erfindung ist eine Verwendung einer kosmetischen Zusammensetzung enthaltend, in einem kosmetisch akzeptablen Medium, mindestens ein Haar-Konditionierungsmittel ausgewählt aus der Gruppe von kationischen Polymeren, kationischen Tensiden, nicht polymer vorliegenden quaternären Ammoniumverbindungen, Organopolysiloxanen und Organopolysiloxancopolymeren, die von den vorvernetzten Organopolysiloxanen der Formel (I) verschieden sind, Fettsäureestern und Fettsäurealkoholen, natürlichen oder synthetischen Ölen und Wachsen und Panthenol, Lipiden, Proteinen und hydrolysierten Proteinen, sowie deren Mischungen und mindestens eine wässrige Dispersion von vorvernetzten Organopolysiloxanen aus Einheiten der allgemeinen Formel

$$AKT_n R^2{}_b R_c (OR^1)_d SiO_{\frac{4-(n+b+c+d)}{2}} \qquad (I)$$

wobei

R      gleich oder verschieden sein kann und einen einwertigen, gesättigten oder ungesättigten Kohlenwasserstoffrest mit 1 bis 200 Kohlenstoffatomen je Rest, der ein oder mehrere Heteroatome aus der Gruppe N, P, S, O, und Halogen enthalten kann, bedeutet,

$R^1$ gleich oder verschieden sein kann, ein Wasserstoffatom oder einen Alkylrest mit 1 bis 8 Kohlenstoffatomen bedeutet, der durch ein oder mehrere separate Sauerstoffatome unterbrochen sein kann,

$R^2$ gleich oder verschieden sein kann, einen einwertigen, gegebenenfalls substituierten Kohlenwasserstoffrest mit 1 bis 18 Kohlenstoffatomen je Rest bedeutet,

AKT ein Rest der Formol $-CH_2NHR^4$, $-CH_2NR^4_2$ oder $-CH_2NR^5$ ist, wobei

$R^4$ einen einwertigen, Kohlenwasserstoffrest mit 1 bis 18 Kohlenstoffatomen, der ein oder mehrere Heteroatome aus der Gruppe N und O enthalten kann, bedeutet und

$R^5$ einen zweiwertigen Kohlenwasserstoffrest mit 3 bis 12 Kohlenstoffatomen, der ein oder mehrere Heteroatome aus der Gruppe N und O enthalten kann, bedeutet.

b 0, 1 oder 2, vorzugsweise 0 oder 1, ist,

c 0, 1, 2 oder 3 ist

d 0, 1 oder 2 ist und

n 0, 1 oder 2 ist,

mit der Maßgabe, dass die Summe $n+b+c+d \leq 3$ ist und dass durchschnittlich je Molekül mindestens ein Rest AKT enthalten ist,

zur Behandlung von keratinischen Fasern, bevorzugt Haaren.

Wässrige Dispersionen von vorvernetzten Organopolysiloxanen und deren Herstellung sind in US 2008/0064813 A1 beschrieben.

Die wässrige Dispersion von vorvernetzten Organopolysiloxanen wird vorzugsweise erhalten, indem mindestens ein Organopolysiloxan (1) aus Einheiten der allgemeinen Formel

$$R_c(OR^1)_d SiO_{\frac{4-(c+d)}{2}} \qquad (II)$$

wobei R und $R^1$ die oben dafür angegebene Bedeutung haben, c 0, 1, 2 oder 3 und d 0, 1 oder 2 ist,

mit der Maßgabe, dass die Summe $c+d \leq 3$ ist und im Organopolysiloxan (1) durchschnittlich mindestens ein Rest $OR^1$ je Molekül, bevorzugt in der Bedeutung von $R^1$ gleich Wasserstoffatom, enthalten ist,

mit mindestens einem hochreaktiven Silan (2) der allgemeinen Formel

$$(AKT)_a R^2_b Si(OR^3)_{4-(a+b)} \qquad (III)$$

oder deren Teilhydrolysate

wobei AKT und $R^2$ die oben dafür angegebene Bedeutung haben,

$R^3$ einen Alkylrest mit 1 bis 8 Kohlenstoffatomen je Rest bedeutet,

a 1 oder 2, bevorzugt 1, ist und

b 0, 1 oder 2, bevorzugt 0 oder 1 ist,

mit der Maßgabe, dass die Summe $a+b \leq 3$ ist,

in Gegenwart von Wasser (3),

und Emulgator (4)

umgesetzt wird,

mit der Maßgabe, dass keine metallhaltigen Katalysatoren mit verwendet werden und

dass Organopolysiloxan (1) und Silan (2) in Art und Menge so gewählt werden, dass die Organopolysiloxane in den erhaltenen Dispersionen vorvernetzt sind.

Bei der Herstellung der erfindungsgemäßen Dispersionen können gegebenenfalls weitere Stoffen, die an der Umsetzung nicht direkt teilnehmen, eingesetzt werden.

Die erfindungsgemäßen Dispersionen enthalten daher vorzugsweise vorvernetzte Organopolysiloxane bestehend aus Einheiten der Formel (I)

Wasser (3)

Emulgator (4)

Die erfindungsgemäßen Dispersionen enthalten daher vorzugsweise keine Katalysatoren.

Bei der Herstellung der erfindungsgemäßen Dispersionen werden Organopolysiloxan (1) und Silan (2) in Art und Menge so gewählt, dass die Organopolysiloxane in den erhaltenen Dispersionen vorvernetzt sind.

Die erfindungsgemäßen Dispersionen enthalten daher bereits vorvernetzte Organopolysiloxane, die nach dem Entfernen des Wassers weiter vernetzen und vorzugsweise elastomere Filme bilden, die bevorzugt in Toluol unlöslich sind, wobei die vernetzten Organopolysiloxane hochmolekulare verzweigte oder dendrimerartige hochverzweigte Strukturen aufweisen.

In Toluol unlöslich wird dabei folgendermaßen definiert:

Das elastomere Material, das nach Eindampfen der Emulsion über einen Zeitraum von 48 h bei 25°C verbleibt, wird als in Toluol unlöslich definiert, wenn 3 g des durch Eindampfen erhaltenen elastomeren Materials in 7 g Toluol nicht löslich sind.

Dies steht im Gegensatz zu unvernetzten Organopolysiloxanen, die auch hochviskos sein können, die aber in Toluol löslich sind.

Vorzugsweise sind die erfindungsgemäßen Dispersionen wässrige Suspensionen oder wässrige Emulsionen von vorvernetzten Organopolysiloxanen.

Die erfindungsgemäßen Dispersionen bilden beim Eintrocknen - ohne Katalysatorzugabe oder Veränderung des pH-Wertes - ein Siliconnetzwerk, vorzugsweise ein elastisches Siliconnetzwerk aus.

Bei der Herstellung der erfindungsgemäßen vorvernetzten Organopolysiloxane werden vorzugsweise endständige OH-Gruppen enthaltende Polyorganosiloxane und schnell reagierende Vernetzer benötigt, und diese Komponenten reagieren vorzugsweise bei Raumtemperatur miteinander. Zur Unterstützung dieser Reaktion werden keine metallhaltigen, zusätzlichen Katalysatoren benötigt, d.h. es werden vorzugsweise keine Übergangsmetalle der VIII. Nebengruppe des Periodensystems und deren Verbindungen und keine Metalle der III., IV. und V. Hauptgruppe des Periodensystems und deren Verbindungen verwendet, wobei die Elemente C, Si, N, und P in dieser Definition nicht als Metalle gelten.

Die Reaktion verläuft ferner vorzugsweise im neutralen Bereich, d.h. im pH-Bereich von ca. 5 bis 8, der sich durch die Komponenten selbst ergibt. Durch die hohe Reaktivität ist ferner eine gezielt geführte chemische Umsetzung nicht nötig und vorzugsweise auch kein Erwärmen.

Vorzugsweise ist R ein einwertiger, gesättigter oder ungesättigter Kohlenwasserstoffrest mit 1 bis 18 Kohlenstoffatomen, der gegebenenfalls ein oder mehrere Heteroatome aus der Gruppe N, P, S, O, und Halogen enthalten kann

Beispiele für Kohlenwasserstoffreste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 5-Hexenyl-, Cyclohexenyl-, 1-Propenyl-, Allyl-, 3-Butenyl- und 4-Pentenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der $\alpha$-und der $\beta$-Phenylethylrest.

Bevorzugt sind als Rest R der Methyl-, Ethyl-, Octyl- und Phenylrest, besonders bevorzugt sind der Methyl- und Ethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Weitere Beispiele für substituierte Reste R sind alle für R oben genannten Reste, die ein oder mehrere N-Atome enthalten können. Bevorzugte Beispiele für substituierte Reste R sind daher Aminoreste A der allgemeinen Formel

$$-R^6-[NR^7-R^8-]_z NR^7_2,$$

wobei $R^6$ ein zweiwertiger linearer oder verzweigter Kohlenwasserstoffrest mit 3 bis 18 Kohlenstoffatomen, vorzugsweise ein Alkylenrest mit 3 bis 10 Kohlenstoffatomen, bedeutet,

$R^7$ ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^8$ ein zweiwertiger Kohlenwasserstoffrest mit 1 bis 6 Kohlenstoffatomen, vorzugsweise ein Alkylenrest mit 1 bis 6 Kohlenstoffatomen, bedeutet,

z 0, 1, 2, 3 oder 4, vorzugsweise 0 oder 1 ist.

Ein weiteres Beispiel für substituierte Reste R sind Polyetherreste **E** der allgemeinen Formel

$$-CH_2CH_2(CH_2)_u O(C_2H_4O)_v(C_3H_6O)_w R^9,$$

wobei

$R^9$ einen Kohlenwasserstoffrest mit 1 bis 6 Kohlenstoffatomen bedeutet,

u 0 oder eine ganze Zahl von 1 bis 16, vorzugsweise 1 bis 4, ist,

v 0 oder eine ganze Zahl von 1 bis 35, vorzugsweise 1 bis 25, ist und

w 0 oder eine ganze Zahl von 1 bis 35, vorzugsweise 1 bis 25 ist,

mit der Maßgabe, dass die Summe v+w 1 bis 70, vorzugsweise 1 bis 50 ist.

Beispiele für Rest $R^1$ sind die vorstehend bei R aufgeführten Alkylreste sowie der Methoxyethyl- , der Ethoxyethylrest und der Hexoxyethylrest, wobei es sich bei dem Rest $R^1$ bevorzugt um Wasserstoff und den Methyl- und den Ethylrest handelt.

Beispiele für Kohlenwasserstoffreste R gelten in vollem Umfang für Reste $R^2$.

Bevorzugte Beispiele für Reste $R^3$ sind der Methyl- und Ethylrest, besonders bevorzugt der Ethylrest.

Beispiele für Kohlenwasserstoffreste R, wie Alkyl-, Cycloalkyl-, Aryl-, Alkaryl- und Aralkylreste R, gelten im vollen Umfang für Kohlenwasserstoffreste $R^4$, wobei Reste der Formel

$-CH_3$

$-CH_2-CH_3$

$-CH(CH_3)-CH_3$

$-CH_2-CH_2-CH_2-CH_3$

$-CH_2-CH(CH_3)-CH_3$

-Cyclohexyl und

-Phenyl

bevorzugt sind.

Beispiele für Kohlenwasserstoffreste $R^4$, die N- und/oder O-Atome enthalten, sind

$-(CH_2)_2N(CH_3)_2$

$-(CH_2)_3N(CH_3)_2$

-Phenyl-$NH_2$

-Phenyl-$OCH_3$

Beispiele für zweiwertige Kohlenwasserstoffreste $R^5$ sind Alkylenreste mit 3 bis 12 Kohlenstoffatomen.

Beispiele für Kohlenwasserstoffreste $R^5$, die N- und/oder 0-Atome enthalten, sind Reste der Formel

$-CH_2-CH_2-O-CH_2-CH_2-$

$-CH_2-CH_2-NH-CH_2-CH_2-$ und

$-CH_2-CH_2-NH-CH_2-$ .

Ein bevorzugtes Beispiel für $R^5$ ist der Rest der Formel $-CH_2-CH_2-O-CH_2-CH_2-$.

Beispiele für Kohlenwasserstoffreste $R^9$ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest, Hexylreste, wie der n-Hexyl- und der iso-Hexylrest.

Bevorzugt als Rest $R^9$ ist ein Methylrest.

Vorzugsweise werden bei der Herstellung der erfindungsgemäßen Dispersionen als Organopolysiloxane (1) Siloxane der allgemeinen Formel

$$(R^1O)R_2SiO(SiR_2O)_eSiR_2(OR^1) \qquad (IVa)$$

eingesetzt, wobei R und $R^1$ die oben dafür angegebene Bedeutung haben und

e eine ganze Zahl von 1 bis 1000 ist.

Bevorzugt werden bei der Herstellung der erfindungsgemäßen Dispersionen als Organopolysiloxane (1) Siloxane der allgemeinen Formel

$$(R^1O)R'_2SiO(SiR'_2O)_eSiR'_2(OR^1) \qquad (IVb)$$

eingesetzt, wobei $R^1$ und e die oben dafür angegebene Bedeutung haben und

R' gleich oder verschieden sein kann, ein einwertiger Kohlenwasserstoffrest mit 1 bis 18 Kohlenstoffatomen bedeutet, mit der Maßgabe, dass 25 bis 100 %, vorzugsweise 50 bis 100 % aller Reste $R^1$ Wasserstoffatome sind.

Besonders bevorzugt werden bei der Herstellung der erfindungsgemäßen Dispersionen als Organopolysiloxane (1) Aminöle, also Siloxane der allgemeinen Formel

$$(R^1O)R'_2SiO(SiAR'O)_p(SiR'_2O)_qSiR'_2(OR^1) \qquad (IVc)$$

eingesetzt, wobei R' und $R^1$ die oben dafür angegebene Bedeutung haben,

der Aminorest A die oben dafür angegebene Bedeutung hat,

p eine ganze Zahl von 1 bis 1000, vorzugsweise 1 bis 10 und q 0 oder eine ganze Zahl von 1 bis 2000, vorzugsweise 50 bis 1000, ist,

mit der Maßgabe, dass 10 bis 100 %, vorzugsweise 20 bis 100 % aller Reste $R^1$ Wasserstoffatome sind.

Im Rahmen dieser Erfindung soll Formel (IVc) so verstanden werden, dass p Einheiten -(SiAR'O)- und q Einheiten -(SiR'$_2$O)-in beliebiger Weise, beispielsweise als Block oder statistisch, im Organopolysiloxanmolekül verteilt sein können.

Bevorzugt sind daher wässrige Dispersionen von vorvernetzten Organopolysiloxanen aus Einheiten der allgemeinen Formel

$$AKT_n R^2{}_b A_m R'{}_c (OR^1)_d SiO_{\frac{4-(n+m+b+c+d)}{2}} \qquad (I')$$

wobei AKT, A, R' , $R^1$, $R^2$, b, c, d und n die oben dafür angegebene Bedeutung haben,

m 0 oder 1 ist,

mit der Maßgabe, dass die Summe n+m+b+c+d $\leq$ 3 ist und n und m in derselben Siloxaneinheit nicht gleichzeitig 1 sind und dass durchschnittlich je Molekül mindestens ein Rest AKT und ein Rest A enthalten ist.

Beispiele für Kohlenwasserstoffreste R gelten im vollen Umfang für Kohlenwasserstoffreste R'.

Beispiele für Reste A sind:

-(CH$_2$)$_3$NH$_2$

-(CH$_2$)$_3$-NH-(CH$_2$)$_2$-NH$_2$

-CH$_2$CH(CH$_3$)CH$_2$-NH-(CH$_2$)$_2$-NH$_2$

-(CH$_2$)$_3$-NH(Cyclohexyl)

-(CH$_2$)$_3$-NHCH$_3$

-(CH$_2$)$_3$-N(CH$_3$)$_2$

-(CH$_2$)$_3$-NHCH$_2$CH$_3$

-(CH$_2$)$_3$-N(CH$_2$CH$_3$)$_2$

-(CH$_2$)$_4$-NH$_2$

-CH$_2$CH(CH$_3$)CH$_2$-NH$_2$

- (CH$_2$)$_3$-NH-(CH$_2$)$_2$-NHCH$_3$

- (CH$_2$)$_3$-NH-(CH$_2$)$_2$-N(CH$_3$)$_2$

- (CH$_2$)$_3$-NH-(CH$_2$)$_2$-NHCH$_2$CH$_3$

- (CH$_2$)$_3$-NH-(CH$_2$)$_2$-N(CH$_2$CH$_3$)$_2$

- (CH$_2$)$_3$[-NH-CH$_2$CH$_2$]$_2$-NH$_2$

und deren teil- oder vollacetylierte Formen, wie

- (CH$_2$)$_3$-NH(Acetyl)

- (CH$_2$)$_3$-NH-(CH$_2$)$_2$-NH(Acetyl) und

- (CH$_2$)$_3$-N(Acetyl)-(CH$_2$)$_2$-NH(Acetyl).

Bevorzugte Beispiele für Reste A sind:

- (CH$_2$)$_3$NH$_2$

- (CH$_2$)$_3$-NH-(CH$_2$)$_2$-NH$_2$

- CH$_2$CH(CH$_3$)CH$_2$-NH-(CH$_2$)$_2$-NH$_2$

- (CH$_2$)$_3$-NHCH$_3$

-(CH$_2$)$_3$-NH(Cyclohexyl)

- (CH$_2$)$_3$-NH(Acetyl)

- (CH$_2$)$_3$-NH-(CH$_2$)$_2$-NH(Acetyl) und

- (CH$_2$)$_3$-N(Acetyl)-(CH$_2$)$_2$-NH(Acetyl).

Bei der Herstellung der erfindungsgemäßen Dispersionen können als Organopolysiloxane (1) auch solche Siloxane (Harze) der allgemeinen Formel

$$[(R_3SiO_{1/2})_f(R_2SiO_{2/2})_g(RSiO_{3/2})_h(SiO_{4/2})_k] \qquad (V)$$

eingesetzt werden, wobei R die oben dafür angegebene Bedeutung hat und zusätzlich R in Formel (V) auch gleich (OR$^1$)

sein kann mit der oben dafür angegebenen Bedeutung, mit der Maßgabe, dass mindestens ein Rest -OR$^1$, wobei R$^1$ ein Wasserstoffatom bedeutet, je Molekül enthalten ist,

f, g, h und k eine ganze Zahl von 0 bis 1000 und h/(f+g+h+k) bevorzugt > 0,2 ist.

Beispiele für Siloxane (1) sind handelsübliche Polydimethylsiloxane mit endständigen Silanolgruppen und Polydimethylsiloxane mit endständigen Alkoxy- und Silanolgruppen.

Weitere Beispiele für Siloxane (1) sind handelsübliche funktionalisierte Siloxane, wie Aminöle, z.B. Aminöle mit 3-(2-Aminoethyl)aminopropylfunktionen, Glykolöle, Phenyl- oder Phenylmethylöle, die Silanolgruppen oder Alkoxy- und Silanolgruppen enthalten.

Weitere Beispiele für Siloxane (1) sind harzartige Siloxane, z.B. Methylsiliconharze, mit 80 Mol% CH$_3$SiO$_{3/2}$ und 20 Mol% (CH$_3$)$_2$SiO$_{2/2}$ und einer Molmasse von ca. 5000g/Mol oder 98 Mol% CH$_3$SiO$_{3/2}$ und 2 Mol% (CH$_3$)$_2$SiO$_{2/2}$ und einer Molmasse von ca. 5000g/Mol, oder z.B. Methylphenylsiliconharze mit 65 Mol% C$_6$H$_5$SiO$_{3/2}$ und 35 Mol% (CH$_3$)$_2$SiO$_{2/2}$, deren restliche freie Valenzen R$^1$O-Gruppen der o.g. Bedeutung tragen.

Bei der Herstellung der erfindungsgemäßen Dispersionen kann eine Art von Organopolysiloxan (1) oder verschiedene Arten von Organopolysiloxan (1) eingesetzt werden.

Die bei der Herstellung der erfindungsgemäßen Dispersionen eingesetzten Organopolysiloxane (1) weisen vorzugsweise Viskositäten von 1 mPa.s bis 50.000.000 mPa.s bei 25°C, bevorzugt 50 mPa.s bis 10.000.000 mPa.s bei 25°C und besonders bevorzugt 100 mPa.s bis 500.000 mPa.s bei 25°C auf

Bei der Herstellung der erfindungsgemäßen Dispersionen kann eine Art von Silan(2) oder verschiedene Arten von Silan (2) eingesetzt werden.

Beispiele für Reste AKT sind der Aminomethyl-, Methylaminomethyl-, Dimethylaminomethyl-, Diethylaminomethyl-, Dibutylaminomethyl-, Cyclohexylaminomethyl-, Morpholinomethyl-, Piperidinomethyl- und Piperazinomethylrest, wobei der Cyclohexylaminomethyl-und Morpholinomethylrest bevorzugt sind.

Beispiele für Silane (2) sind Diethylaminomethyl-methyldimethoxysilan, Dibutylaminomethyltriethoxysilan, Dibutylaminomethyltributoxysilan, Cyclohexylaminomethyltrimethoxysilan, Cyclohexylaminomethyltriethoxysilan, Cyclohexylaminomethyl-methyldiethoxysilan, Anilinomethyltriethoxysilan, Anilinomethyl-methyldiethoxysilan, Morpholinomethyltriethoxysilan, Morpholinomethyltrimethoxysilan, Morpholinomethyltriisopropoxysilan, 3-Dimethylaminopropyl-aminomethyltrimethoxysilan, Morpholinomethyltributoxysilan, Morpholinomethyltrialkoxysilan, wobei der Alkoxyrest ein C$_1$-C$_4$-Alkoxyrest ist, insbesondere ein Gemisch aus Methoxy- und Ethoxyrest ist, Piperazinomethyltriethoxysilan und Piperidinomethyltriethoxysilan

Bevorzugt sind dabei Silane (2), die eine Trialkoxy-Gruppe tragen, d.h. bei denen b in Formel (III) 0 ist.

Die vorvernetzten Organopolysiloxane können je nach Einsatz von Di- oder Trialkoxysilan (2) oder Alkoxy- und Hydroxygruppen tragendem Teilhydrolysat von (2) und linearem, verzweigtem oder harzartigem Siloxan (1) verzweigte oder sogar hochverzweigte/stark vernetzte Strukturen mit linearen Anteilen haben.

Werden Dialkoxysilane (2) mit rein linear aufgebauten Siloxanen (1), die maximal 2 SiOH-Funktionen pro Molekül enthalten, insbesondere mit den Siloxanen der Formeln (IVa) oder (IVb), umgesetzt, werden lineare hochviskose Organopolysiloxane, und keine erfindungsgemäßen vernetzten Organopolysiloxane erhalten. Die Umsetzung von Siloxanen (1), die mehr als 2 OH-Funktionen, insbesondere mindestens 3 OH-Funktionen, pro Molekül enthalten, mit Dialkoxysilanen (2) führt dagegen zu vernetzten Siloxanpolymeren.

Werden als Silane (2) Trialkoxysilane eingesetzt, was bevorzugt ist, werden erfindungsgemäße vorvernetzte Organopolysiloxane erhalten. Ferner werden auch bei Einsatz von Gemischen aus Dialkoxysilanen (2) und Trialkoxysilanen (2), insbesondere bei Einsatz von Gemischen aus 1 - 99 Gew.% Dialkoxysilanen (2) und 1 - 99 Gew.% Trialkoxysilanen (2), bevorzugt 10 - 90 Gew.% Dialkoxysilanen (2) und 10 - 90 Gew.% Trialkoxysilanen (2), erfindungsgemäße vorvernetzte Organopolysiloxane erhalten.

Der Vernetzungsgrad hängt dabei vom eingesetzten Verhältnis der Äquivalente -OR$^3$ in Silan (2) zu -OR$^1$ in Siloxan (1) ab.

Zur Herstellung der erfindungsgemäßen Dispersionen aus Siloxan (1) und hochreaktivem Silan (2) wird Silan (2) oder dessen Teilhydrolysate dabei vorzugsweise in Mengen von 0,6 bis 5 Äquivalente -OR$^3$, bevorzugt 0,65 bis 2 Äquivalente -OR$^3$, besonders bevorzugt 0,7 bis 1,5 Äquivalente -OR$^3$, je Äquivalent -OR$^1$ in Siloxan (1) eingesetzt, wobei R$^1$ bevorzugt ein Wasserstoffatom ist.

Die Herstellung der erfindungsgemäßen Dispersionen von vorvernetzten Organopolysiloxane erfolgt durch intensives Mischen von Siloxanen aus der Gruppe der Siloxane (1) mit Silanen (2)

Wasser (3), und

Emulgatoren (4) miteinander. Die Herstellung kann diskontinuierlich oder kontinuierlich erfolgen.

Obwohl die Silane (2) bekanntermaßen hydrolyseempfindliche Gruppen enthalten, besonders wenn R$^3$ ein Methyl- oder Ethylist, werden selbst in Gegenwart von Wasser überraschenderweise vorvernetzte Organopolysiloxane durch Reaktion mit Siloxanen (1) erhalten.

Die Art der Mischung der Komponenten, die zur Herstellung der erfindungsgemäßen Dispersionen gebraucht werden, ist nicht sehr kritisch und kann in verschiedener Reihenfolge ausgeübt werden. In Abhängigkeit von den Komponenten (1), (2), (3) und (4) können sich aber bevorzugte Vorgehensweisen ergeben, die im Einzelfall geprüft werden sollten.

Es können z.B. die Komponenten (1) und (2) miteinander vorgemischt werden, daraufhin der (oder die) Emulgator(en) zugefügt und danach Wasser (3) eingearbeitet wird. Es ist auch möglich, die Komponenten (1) bis (4) der Reihe nach in die Emulgierapparatur zu dosieren. In besonderen Fällen kann es z.B. aufgrund der Siloxanviskosität oder -reaktivität vorteilhaft sein, Silan (2) mit einem Siloxan (1) zu mischen und danach ein anderes Siloxan (1) einzuarbeiten, oder umgekehrt, je nachdem, wie sich günstigere rheologische Eigenschaften für die Verarbeitung der Komponenten ergeben. Bei sehr reaktiven Silanen (2) kann es vorteilhaft sein, erst die Komponente (1) mit Emulgator (4) und Wasser (3) in eine steife Phase überzuführen, und danach das Silan (2) pur oder verdünnt in einem inerten Stoff einzudosieren, bevor eine Phasenumkehr und damit z.B. eine Öl-in-Wasser-Dispersion erhalten wird.

Desweiteren ist es auch möglich, Silan (2) in die fertige Emulsion von Siloxanen (1) zu geben, um so die gewünschte Reaktion und Vernetzung des Siloxan (1) in der Emulsion zu erreichen. Ferner kann das Silan (2) zuvor durch Zugabe von Wasser teilweise oder vollständig hydrolysiert werden. Um VOCfreies Hydrolysat von (2) zu erhalten kann das Nebenprodukt Alkohol $R^3OH$ durch geeignete bekannte Maßnahmen wie Destillation, Membranverfahren oder andere Trennverfahren teilweise oder vollständig entfernt werden.

Bei der Herstellung der erfindungsgemäßen Dispersionen wird Wasser (3), in Mengen von vorzugsweise 1 bis 99 Gew.-%, besonders bevorzugt 25 bis 95 Gew.-%, jeweils bezogen auf das Gesamtgewicht aller Inhaltsstoffe der Dispersion eingesetzt.

Die erfindungsgemäße wässrige Dispersion von vorvernetzten Organopolysiloxanen wird als Öl in Wasser System eingesetzt.

Als Emulgatoren (4) zur Herstellung der wässrigen Dispersionen von vorvernetzten Organopolysiloxanen können alle bisher bekannten, anionische, nichtionische, kationische oder amphotere Emulgatoren sowohl einzeln als auch als Mischungen verschiedener Emulgatoren verwendet werden, mit denen auch bisher wässrige Dispersionen, insbesondere wässrige Emulsionen von Organopolysiloxanen hergestellt werden konnten.

Beispiele für anionische Emulgatoren sind:

1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 40 Ethylenoxid (EO)- bzw. Propylenoxid(PO)einheiten.

2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 40 EO-Einheiten ethoxyliert sein.

3. Alkali- und Ammoniumsalze von Carbonsäuren mit 8 bis 20 C-Atomen im Alkyl-, Aryl-, Alkaryl- oder Aralkylrest.

4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.

Beispiele für nichtionische Emulgatoren sind:

5. Polyvinylalkohol, der noch 5 bis 50 %, vorzugsweise 8 bis 20 % Vinylacetateinheiten aufweist, mit einem Polymerisationsgrad von 500 bis 3000.

6. Alkylpolyglycolether, vorzugsweise solche mit 3 bis 40 EO-Einheiten und Alkylresten von 8 bis 20 C-Atomen.

7. Alkylarylpolyglycolether, vorzugsweise solche mit 5 bis 40 EO-Einheiten und 8 bis 20 C-Atomen in den Alkyl- und Arylresten.

8. Ethylenoxid/Propylenoxid(EO/PO)-Blockcopolymere, vorzugsweise solche mit 8 bis 40 EO- bzw. PO-Einheiten.

9. Additionsprodukte von Alkylaminen mit Alkylresten von 8 bis 22 C-Atomen mit Ethylenoxid oder Propylenoxid.

10. Fettsäuren mit 6 bis 24 C-Atomen.

11. Alkylpolyglykoside der allgemeinen Formel $R''\text{-}O\text{-}Z_O$, worin $R''$ einen linearen oder verzweigten, gesättigten oder ungesättigten Alkylrest mit im Mittel 8 - 24 C-Atomen und $Z_O$ einen Oligoglykosidrest mit im Mittel o = 1 - 10 Hexose- oder Pentoseeinheiten oder Gemischen davon bedeuten.

12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.

13. Polare Gruppen, enthaltend insbesondere die Elemente O, N, C, S, P, Si, enthaltende lineare Organo(poly)siloxane, insbesondere solche mit Alkoxygruppen mit bis zu 24 C-Atomen und/oder bis zu 40 EO- und/oder PO-Gruppen.

Beispiele für kationische Emulgatoren sind:

14. Salze von primären, sekundären und tertiären Fettaminen mit 8 bis 24 C-Atomen mit Essigsäure, Schwefelsäure, Salzsäure und Phosphorsäuren.

15. Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate.

16. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Als amphotere Emulgatoren eignen sich besonders:

17. Langkettig substituierte Aminosäuren, wie N-Alkyl-di-(aminoethyl-)glycin oder N-Alkyl-2-aminopropionsäuresalze.

18. Betaine, wie N-(3-Acylamidopropyl)-N,N-dimethylammoniumsalze mit einem $C_8$-$C_{18}$-Acylrest und Alkyl-imidazolium-Betaine oder quaternisierte Alkyl oder substituierte Alkylderivate des N,N-Dimethyl-Glycins.

Bevorzugt als Emulgatoren zur Herstellung für wässrige Dispersionen vorvernetzter Organopolysiloxane sind nichtionische Emulgatoren, insbesondere die vorstehend unter 6. aufgeführten Alkylpolyglycolether

Der Bestandteil (4) kann aus einem der o.g. Emulgatoren oder aus einem Gemisch zweier oder mehrerer o.g. Emulgatoren bestehen, er kann in reiner Form oder als Lösungen eines oder mehrerer Emulgatoren in Wasser oder organischen Lösungsmitteln eingesetzt werden.

Bei der Herstellung der erfindungsgemäßen Dispersionen werden die Emulgatoren (4) in Mengen von vorzugsweise 0,1 bis 60 Gew.-%, besonders bevorzugt 0,5 bis 30 Gew.-%, jeweils bezogen auf das Gesamtgewicht an Siloxanen (1) und Silane (2), eingesetzt.

Falls das Organopolysiloxan (1) oder das Silan (2) bzw. das entstehende vorvernetzte Organopolysiloxan selbst als Emulgator wirkt, kann auf den Zusatz von separatem Emulgator (4) verzichtet werden.

Der Emulgiervorgang zur Herstellung der Dispersion wird vorzugsweise bei Temperaturen unter 120°C, bevorzugt bei 5°C bis 100°C, besonders bevorzugt bei 10°C bis 80°C durchgeführt. Die Temperaturerhöhung kommt vorzugsweise durch den Eintrag mechanischer Scherenergie, die für den Emulgierprozess benötigt wird, zustande. Die Temperaturerhöhung wird nicht zur Beschleunigung eines chemischen Prozesses benötigt. Weiterhin erfolgt die Herstellung der Dispersionen vorzugsweise beim Druck der umgebenden Atmosphäre, sie kann aber auch bei höheren oder niederen Drücken durchgeführt werden.

Die Umsetzung von (1) mit (2) bei der Herstellung der Dispersionen läuft vorzugsweise in wenigen Minuten bis mehreren Stunden vollständig ab, wobei Methoxysilane schneller als Ethoxysilane reagieren.

Die bei der Herstellung der Dispersionen als Kondensationsnebenprodukte anfallenden Alkohole können im Produkt verbleiben oder auch entfernt werden, beispielsweise durch Destillation unter Vakuum, Membranverfahren, oder durch Extraktion.

Die mittels Lichtstreuung in den erfindungsgemäßen Dispersionen gemessene mittlere Teilchengröße liegt vorzugsweise im Bereich 0,001 bis 50 $\mu$m, bevorzugt bei 0,005 bis 10 $\mu$m, besonders bevorzugt im Bereich 0,01 bis 5 $\mu$m. Die pH-Werte können von 1 bis 14 variieren, bevorzugt 3 bis 9, besonders bevorzugt 4 bis 8.

Die erfindungsgemäße kosmetische Zusammensetzung enthält wässrige Dispersionen von vorvernetzten Organopolysiloxanen vorzugsweise in Mengen von 0,2 bis 40 Gew.-%, bevorzugt von 0,5 bis 30 Gew.-%, insbesondere bevorzugt 1 bis 20 Gew.-%, jeweils bezogen auf das Gesamtgewicht der kosmetischen Zusammensetzung.

Bevorzugt ist in der erfindungsgemäßen kosmetischen Zusammensetzung das kosmetisch akzeptable Medium Wasser.

Die erfindungsgemäße kosmetische Zusammensetzung enthält mindestens ein Haar-Konditionierungsmittel, im folgenden einfach als Konditionierungsmittel genannt. Als Konditionierungsmittel werden analog zu K. Krummel, Stephane Chiron, J. Jachowicz, Chapter 14, in: "The Chemistry and Manufacture of Cosmetics", Volume II, Formulating, Third Edition by Mitchell L. Schlossmann, 2000, S. 359-396, kosmetische Inhaltsstoffe bezeichnet, welche die Haaroberfläche modifizieren und die Beschaffenheit des Haares beeinflussen. Kosmetische Zusammensetzungen enthaltend Konditionierungsmittel werden eingesetzt zur Modifizierung oder Verbesserung der Weichheit der Haare, besseren Entwirrbarkeit, Verringerung der Nass- und Trockenkämmkraft, Pflege der Haare, Vermeidung elektrostatischer Aufladung, leichterer Gleitwirkung durch das Haar und entlang der Haaroberfläche, Verbesserung des Haarglanzes, Erhalt der Farbechtheit von Haaren, Verringerung des Haarbruchs, Erhalt der Haarform und weiterer kosmetischer Eigenschaften, die mit natürlichem und gesundem Haar in Verbindung gebracht werden.

Die erfindungsgemäße kosmetische Zusammensetzung verbessert einen oder mehrere der oben genannten Effekte.

Beispiele für Konditionierungsmittel und deren INCI-Namen sind beschrieben im "International Cosmetic Ingredient Dictionary & Handbook" des Personal Care Product Council (Hrsg.).

Als Referenz kann das World Wide Web basierte "wINCI Web Based International Cosmetic Ingredient Dictionary & Handbook (http://online.personalcarecouncil.org/jsp/Home.jsp) oder das International Cosmetic Ingredient Dictionary & Handbook, 13th Edition, The Personal Care Products Council (formerly: The Cosmetic, Toiletry, and Fragrance Association (CTFA)), 2010, Verwendung finden.

Bevorzugte Beispiele für Konditionierungsmittel sind kationische Polymere. Darunter verstanden werden Polymere, die seitenständig oder endständig kationische Gruppen tragen oder seitenständig oder endständig Gruppen, die durch Ionisierung in eine kationische Gruppe überführt werden können.

Vorzugsweise werden kationische Polymere verwendet, die eine quaternäre Ammoniumgruppe aufweisen.

Beispiele für vorzugsweise eingesetzte kationische Polymere sind im International Cosmetic Ingredient Dictionary & Handbook unter der Bezeichnung Polyquaternium veröffentlicht, wobei jedes Polymer mit einem individuellen Zahlenkürzel identifiziert wird, z.B. Polyquaternium-1.

Weitere Beispiele für kationische Polymere sind quaternäre Ammoniumgruppen aufweisende Derivate von modifizierten Polysacchariden, z.B. Polymere mit den INCI-Namen Cassia Hydroxypropyltrimonium Chloride, Derivate von modifizierter Cellulose und/oder Stärke, z.B. ein quaternäres Ammoniumderivat eines mit Propylenglycolether modifizierten Cyamopsis Tetragonoloba (Guar) Gums mit dem INCI-Namen Guar Hydroxypropyltrimonium Chloride, oder polymere quaternäre Ammoniumsalze des Reaktionsprodukts von Hydroxyethylcellulose mit einem Trimethylammonium-substituierten Epoxid, wie Cellulose, 2-hydroxyethyl 2-(2-hydroxy-3-(trimethylammonium)propoxy)ethyl 2-hydroxy-3-(trimethylammonium)propyl ether chlorid, wie Cellulose, 2-hydroxyethyl 2-hydroxy-3-(trimethylammonium)propyl ether, chlorid, wie Cellulose, 2-hydroxyethyl 23-hydroxy-3-(trimethylammonium)propyl ether, chloride, wie Cellulose, 2-[2-hydroxy-3-(trimethylammonium)propoxy]ethyl ether, chloride, mit dem INCI-Namen Polyquaternium-10.

Weitere Beispiele für kationische Polymere sind quaternäre Ammoniumgruppen aufweisende Acrylsäurepolymerderivate, Acrylsäurecopolymerderivate, Methacrylsäurederivate und Methacrylsäurecopolymerderivate, z.B. Polymere mit dem INCI-Namen Polyquaternium-37.

Weitere Beispiele für kationische Polymere sind quaternäre Ammoniumgruppen aufweisende Copolymere aus Dimethyldiallylammoniumchloride and Acrylsäure, z.B. Polmyere mit dem INCI-Namen Polyquaternium-22.

Weitere Beispiele für kationische Polymere sind quaternäre Ammoniumgruppen aufweisende Copolymere aus Derivaten des Vinylpyrrolidon, Viylimidazol und Vinylimidazolin und Methacrylsäure, z.B. Polymere mit dem INCI-Namen Polyquaternium-86.

Weitere Beispiele für kationische Polymere sind quaternäre Ammoniumgruppen aufweisende Copolymere aus Acrylamide and Dimethyl-diallyl-ammoniumchloride, z.B. Polymere mit dem INCI-Namen Polyquaternium-7.

Weitere Beispiele für kationische Polymere sind quaternäre Ammoniumgruppen aufweisende Copolymere aus dem Reaktionsprodukt von Diethylsulfat mit Vinylpyrrolidon und Dimethylaminoethylmethacrylat, z.B. Polmyere mit dem INCI-Namen Polyquaternium-11.

Die erfindungsgemäße kosmetische Zusammensetzung enthält kationische Polymere vorzugsweise in Mengen von 0,01 bis 5 Gew.-%, bevorzugt von 0,05 bis 4 Gew.-%, insbesondere bevorzugt 0,10 bis 3 Gew.-%, jeweils bezogen auf das Gesamtgewicht der kosmetischen Zusammensetzung.

Weitere bevorzugte Beispiele für Konditionierungsmittel sind kationische Tenside. Beispiele für vorzugsweise eingesetzte kationische Tenside entsprechen den in Punkt 14. bis 16. unter Beispiele für kationische Emulgatoren aufgeführten Materialien. Beispiele sind Cetyltrimethylammoniumsalze oder Behenyltrimethylammoniumsalze. Als anionisches Gegenion können beispielsweise Chlorid, Bromid, Methosulfat vorliegen. INCI-Namen von vorzugsweise eingesetzten kationischen Tensiden sind beispielsweise Cetrimonium Chloride, Cetrimonium Methosulfate, Behentrimonium Chloride, Behentrimonium Methosulfate, Steartrimonium Bromide.

Die erfindungsgemäße kosmetische Zusammensetzung enthält kationische Tenside vorzugsweise in Mengen von 0,1 bis 7 Gew.-%, bevorzugt von 0,15 bis 6 Gew.-%, insbesondere bevorzugt 0,2 bis 5 Gew.-%, jeweils bezogen auf das Gesamtgewicht der kosmetischen Zusammensetzung.

Weitere Beispiele für Konditionierungsmittel sind nicht polymer vorliegende quaternäre Ammoniumverbindungen. Darunter verstanden werden nicht polymere Ammoniumverbindungen, die kationisch vorliegen oder durch Ionisierung in eine kationische Gruppe überführt werden können.

Beispiele für vorzugsweise eingesetzte nicht polymer vorliegende quaternäre Ammoniumverbindungen sind Dimethyl Dioctadecyl Ammonium Chloride mit dem INCI-Namen Distearyldimonium Chloride, N-[3-(Dimethylamino)propyl]octadecanamid mit dem INCI-Namen Stearamidopropyl Dimethylamine oder Verbindungen mit dem INCI-Namen Dicocoylethyl Hydroxyethylmonium Methosulfate oder Quaternium-87.

Weitere bevorzugte Beispiele für Konditionierungsmittel sind Organopolysiloxane und Organopolysiloxancopolymere, die von den in den wässrigen Dispersionen vorliegenden vorvernetzten Organopolysiloxanen der Formel (I) verschieden sind. Die Organopolysiloxane können in Form eines Öls, Wachses, Gummis oder Harzes vorliegen oder in Form einer Emulsion.

Beispiele für solche von Organopolysiloxanen der Formel (I) verschiedenen Organopolysiloxane sind:

Cyclische Organopolysiloxane der Formel

$$[R^*_2SiO]_x$$

wobei x eine ganze Zahl von 4 bis 8 ist,

lineare Organopolysiloxane der allgemeinen Formel

$$R^*_3SiO[R^*_2SiO]_ySiR^*_3$$

oder

$$HOSiR^*_2O[R^*_2SiO]_ySiR^*_2OH,$$

wobei y 0 oder eine ganze zahl von 1 bis 2000 ist,

und harzartige Organopolysiloxane der allgemeinen Formel

$$R^*_tSiO_{(4-t)/2}$$

wobei R* jeweils die oben dafür angegebene Bedeutung von R', A oder E hat, und
t 0, 1, 2 oder 3 ist,
so dass das Organopolysiloxanharz aus M, D, T und / oder Q-Einheiten aufgebaut ist, wobei die Kombination vorwiegend oder ausschließlich aus D und T-Einheiten ebenso bevorzugt sind, wie die Kombination vorwiegend oder ausschließlich aus M und Q-Einheiten, wobei im Falle der vorwiegend oder ausschließlich aus D und T-Einheiten aufgebauten Harze T-Einheiten bevorzugt in einem Molverhältnis von T/[M+D+T+Q] von 0,45 bis 1, besonders bevorzugt von 0,55 bis 1,0 vorliegen und die Anzahl der M und Q-Einheiten in beiden Fällen vorzugsweise Null ist und in dem Falle der vorwiegend oder ausschließlich aus M und Q-Einheiten aufgebauten Organopolysiloxanharze Q-Einheiten bevorzugt in einem Molverhältnis von Q/[M+D+T+Q] von 0,25 bis 0,9, besonders bevorzugt von 0,35 bis 0,7 vorliegen und die Anzahl der D und T-Einheiten in beiden Fällen vorzugsweise Null ist.

Beispiele für Organopolysiloxane, vorliegend in Form eines Öls sind Polydimethylsiloxane mit der Viskosität 0,65 bis 2 000 000 mPas (25°C) und den INCI-Bezeichnungen Disiloxan und Dimethicone.

Weitere Beispiele für Organopolysiloxane, vorliegend in Form eines Öls oder Wachses sind funktionalisierte Organopolysiloxane, beispielsweise Polyalkysiloxane, wobei mindestens ein Alkylrest sich unterscheidet von Methyl, beispielsweise Organopolysiloxane mit dem INCI-Namen Stearyl Dimethicone, Cetyl Dimethicone oder C26-28 Alkyl Dimethicone, oder beispielsweise Polyarylsiloxane und Polyarylalkylsiloxane, beispielsweise Organopolysiloxane mit dem INCI-Namen Phenyl Trimethicone, Trimethylsiloxyphenyl Dimethicone oder Dimethylphenyl Dimethicone, oder beispielsweise Organopolysiloxane mit einem organofunktionellen Rest wie einem Aminopropyl-, Aminopropyl-aminoethyl, Aminopropylaminoisobutylrest, beispielsweise Organopolysiloxane mit dem INCI-Namen Amodimethicone, oder beispielsweise Organopolysiloxane mit einem Polyethylenglycol- oder Polyalkylenglycolrest, beispielsweise Organopolysiloxane mit dem INCI-Namen PEG-12 Dimethicone, PEG/PPG-25,25-Dimethicone oder Cetyl PEG/PPG-15/15 Butyl Ether Dimethicone.

Weitere Beispiele für Organopolysiloxane sind Silionharze mit den INCI-Namen Trimethylsiloxysilicate oder Polymethylsilsesquioxane.

Die erfindungsgemäße kosmetische Zusammensetzung enthält Organopolysiloxane und Organopolysiloxancopolymere, die von den in den wässrigen Dispersionen vorliegenden vorvernetzten Organopolysiloxanen der Formel (I) verschieden sind, vorzugsweise in Mengen von 0,1 bis 40 Gew.-%, bevorzugt von 0,2 bis 30 Gew.-%, insbesondere bevorzugt 0,3 bis 20 Gew.-%, jeweils bezogen auf das Gesamtgewicht der kosmetischen Zusammensetzung.

Weitere bevorzugte Beispiele für Konditionierungsmittel sind Fettsäureester und Fettsäurealkohole.

Beispiele für Fettsäurealkohole sind Alkohole mit C8-C28 Kohlenstoffketten wie die Fettalkohole 1-Octadecanol mit dem INCI-Namen Stearyl Alcohol, 1-Hexadecanol mit dem INCI-Namen Cetyl Alcohol, oder Fettalkohole mit den INCI-Namen Cetearyl Alcohol, Myristyl Alcohol, Caprylic Alcohol, Lauryl Alcohol, Decyl Alcohol und Oleyl Alcohol.

Fettsäurealkohole erfüllen neben konditionierenden Eigenschaften auch eine strukturgebende, verdickende Wirkung bei kosmetischen Zusammensetzungen.

Weitere Beispiele für Fettsäureester sind Ester der Fettsäuren mit dem INCI-Namen Palmitic Acid, Oleic Acid, Linolic Acid, Linoleic Acid, Caprylic Acid, Myristic Acid, Stearic Acid, beispielsweise Fettsäureester mit dem INCI-Namen Isopropyl Palmitate, Ethylhexyl Palmitate, Isopropyl Myristate, Isopropyl Stearate.

Die erfindungsgemäße kosmetische Zusammensetzung enthält Fettsäureester und Fettsäurealkohole vorzugsweise in Mengen von 0,1 bis 15 Gew.-%, bevorzugt von 0,3 bis 12 Gew.-%, insbesondere bevorzugt 0,5 bis 10 Gew.-%, jeweils bezogen auf das Gesamtgewicht der kosmetischen Zusammensetzung.

Weitere bevorzugte Beispiele für Konditionierungsmittel sind natürliche oder synthetische Öle und Wachse.

Beispiele für bevorzugte Öle und Wachse sind Kohlenwasserstoffe mit linearen oder verzweigten, gesättigten oder ungesättigten C4-C60 Kohlenstoffketten, wie Öle und Wachse mit den INCI-Namen Isododecane, hydrated Polyisobutylene, hydrated Polydecene, Paraffin und Isoparafinn.

Weitere Beispiele für bevorzugte Öle und Wachse sind Carnaubawachs, Bienenwachs, Wollwachs, mikrokristallines Wachs, Jojobaöl, Reisöl, Calendulaöl, Sonnenblumenöl, Sojaöl, Kokusnussöl, Olivenöl und Mandelöl.

Die erfindungsgemäße kosmetische Zusammensetzung enthält Öle und Wachse vorzugsweise in Mengen von 0,1 bis 10 Gew.-%, bevorzugt von 0,2 bis 7 Gew.-%, insbesondere bevorzugt 0,3 bis 5 Gew.-%, jeweils bezogen auf das Gesamtgewicht der kosmetischen Zusammensetzung.

Weitere bevorzugte Beispiele für Konditionierungsmittel sind Panthenol, Lipide, wie Ceramide, Proteine und hydrolysierte Proteine, wie hydrolisiertes Kollagen, hydrolysierte Weizenproteine und hydrolysierte Seide.

Optional enthält die kosmetische Zusammensetzung weitere kosmetisch übliche Additive, wie z.B. Tenside, Verdicker, Gelierungsmittel, Filmbildner, feuchtigkeitsspendende Mittel, UV-Filter, Perlglanzpigmente, Vitamine, Antioxidantien, Coffein, Anti-Schuppenwirkstoffe oder Konservierungsmittel.

Beispiele für weitere in der Kosmetik übliche Additive und deren INCI-Namen sind beschrieben im "International Cosmetic Ingredient Dictionary & Handbook" des Personal Care Product Council.

Optional enthält die kosmetische Zusammensetzung weitere kosmetisch übliche Additive wie Tenside.

Beispiele für in der Kosmetik übliche Tenside sind auch in K. Schrader, A. Domsch, Cosmetology - Theory and Practice, Volume II, Seite II-8 bis II-22, Verlag für chemische Industrie, 2005, sowie in Punkt 1. bis 18. unter Beispiele für Emulgatoren, beschrieben.

Beispiele für vorzugsweise eingesetzte anionische Tenside entsprechen den in Punkt 1. bis 3. unter Beispiele für anionische Emulgatoren aufgeführten Materialien.

INCI-Namen von vorzugsweise eingesetzten anionische Tensiden sind beispielsweise Sodium Lauryl Sulfate, Ammonium Laureth Sulfate, Sodium Laureth Sulfate, Disodium 2-Sulfolaurate, Disodium Lauryl Sulfosuccinate oder Disodium Laureth-Sulfosuccinate.

Die erfindungsgemäße kosmetische Zusammensetzung enthält anionische Tenside vorzugsweise in Mengen von 1 bis 30 Gew.-%, bevorzugt von 5 bis 25 Gew.-%, insbesondere bevorzugt 7 bis 20 Gew.-%, jeweils bezogen auf das Gesamtgewicht der kosmetischen Zusammensetzung.

Beispiele für vorzugsweise eingesetzte nichtionische Tenside entsprechen den in Punkt 5. bis 13. unter Beispiele für nichtionische Emulgatoren aufgeführten Materialien.

INCI-Namen von vorzugsweise eingesetzten nichtionischen Tensiden sind beispielsweise Coco Glucoside, Lauryl glucoside, Decyl Glucoside, PEG-40 Hydrogenated Castor Oil, Polysorbate 80 oder PEG-7 Glyceryl Cocoate.

Die erfindungsgemäße kosmetische Zusammensetzung enthält nichtionische Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der kosmetischen Zusammensetzung.

Beispiele für vorzugsweise eingesetzte amphotere Tenside entsprechen den in Punkt 17. bis 18. unter Beispiele für nichtionische Emulgatoren aufgeführten Materialien. Weitere bevorzugte Beispiele sind Verbindungen aus den Klassen der Alkylamidobetaine, Alykylamphoacetate und Alkylamphopropionate. INCI-Namen von vorzugsweise eingesetzten nichtionischen Tensiden sind beispielsweise Cocamidopropyl Betaine, Cetyl Betaine, Cocamide MEA, Cocamide DEA, Cocamide MIPA, Sodium Cocoamphoacetate und Sodium Cocoamphopropionate.

Die erfindungsgemäße kosmetische Zusammensetzung enthält amphotere Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der kosmetischen Zusammensetzung.

Optional enthält die kosmetische Zusammensetzung weitere kosmetisch übliche Additive wie Verdicker.

Beispiele für vorzugsweise eingesetzte Verdicker sind modifizierte Polysaccharide wie Stärke, Cellulose, Gummi Arabicum und Guar Gums, z.B. Polmyere mit dem INCI-Namen Cellulose Gum, Guar Gum, Xanthan Gum oder Cassia Gum.

Weitere Beispiele für Verdicker sind hydrophob modifizierte nichtionische Cellulosederivate, z.B. das Cellulosederivat mit dem INCI-Namen Hydroxyethylcellulose.

Weitere Beispiele für Verdicker sind vernetzte Acrylsäure- und Methacrylsäurepolymere und Derivate der vernetzten Acrylsäure- und Methacrylsäurepolymere, z.B. Polymere mit dem INCI-Namen Carbomer.

Weitere Beispiele für Verdicker sind Agentien, die in Kombination mit Tensiden eine verdickende Wirkung erzielen. Beispiele sind Monoglyceride von Fettsäuren, Mono/Diglyceride von ethoxylierten Fettsäuren und ethoxylierte Fettalkohole. INCI-Namen von vorzugsweise eingesetzten Verdickern die in Kombination mit Tensiden eine verdickende Wirkung erzielen sind PEG-120 Methyl Glucose Dioleate, PEG-150 Distearate, Myristyl Glycol, PEG-200 Glyceryl Palmitate, Laureth-4 oder PEG-200 Glyceryl Palmitate.

Weitere Beispiele für Verdicker sind Salze, z.B. Salze mit dem INCI-Namen Sodium Chloride.

Die erfindungsgemäße kosmetische Zusammensetzung enthält Verdicker vorzugsweise in Mengen von 0,1 bis 10 Gew.-%, jeweils bezogen auf das Gesamtgewicht der kosmetischen Zusammensetzung.

Optional enthält die kosmetische Zusammensetzung weitere kosmetisch übliche Additive wie Filmbildner.

Bevorzugte Beispiele für Filmbildner sind Polymere.

Beispiele für vorzugsweise eingesetzte filmbildende Polymere sind beschrieben im "International Cosmetic Ingredient Dictionary & Handbook" des Personal Care Product Councils.

Beispiele für bevorzugte filmbildende Polymere sind Acrylsäurepolymerderivate, Acrylsäurecopolymerderivate, Methacrylsäurederivate und Methacrylsäurecopolymerderivate. Beispiele für bevorzugte anionische Polymere sind Copolymere aus Vinyl Acetate und einem oder mehrerer Acrylsäure-, Methacrylsäuremonomere und deren Ester, z.B Polymere mit dem INCI-Namen Acrylates/VA Copolymer.

Weitere Beispiele für bevorzugte filmbildende Polymere sind Copolymere aus Vinylpyrrolidone und einem oder mehrerer Acrylsäure-, Methacrylsäuremonomere und deren Ester, z.B Polymere mit dem INCI-Namen Acrylates/VP Copolymer.

Weitere Beispiele für bevorzugte filmbildende Polymere sind Copolymere aus tert-Butyl Acrylamide und einem oder mehrerer Acrylsäure-, Methacrylsäuremonomere und deren Ester, z.B Polymere mit dem INCI-Namen Acrylates/t-Butylacrylamide Copolymer.

Weitere Beispiele für bevorzugte filmbildende Polymere sind Copolymere aus Vinyl Acetate, Crotonsäure und Vinyl Neodecanoate Monomeren, z.B. Polymere mit dem INCI-Namen VA/Crotonates/Vinyl Neodecanoate Copolymer.

Weitere Beispiele für bevorzugte filmbildende Polymere sind Copolymere aus Vinyl Acetate, Crotonsäure und Vinyl Neodecanoate Monomeren und Vinylsiliconen, z.B. Polymere mit dem INCI-Namen Crotonic Acid/Vinyl C8-C12 Isoalkyl Esters/VA/Bis-Vinyldimethicone Copolymer.

Die erfindungsgemäße kosmetische Zusammensetzung enthält filmbildende Polymere vorzugsweise in Mengen von 0,1 bis 15 Gew.-%, bevorzugt von 0,2 bis 10 Gew.-%, insbesondere bevorzugt 0,3 bis 7 Gew.-%, jeweils bezogen auf das Gesamtgewicht der kosmetischen Zusammensetzung.

Optional enthält die kosmetische Zusammensetzung weitere kosmetisch übliche Additive wie feuchtigkeitsspendende Mittel.

Beispiele für vorzugsweise eingesetzte feuchtigkeitsspendende Mittel sind Glycerin, Sorbitol, Xylitol, Polyethylenglycol, 1,2-Propandiol, 1,3-Propandiol oder Polypropylenglykol.

Die erfindungsgemäße kosmetische Zusammensetzung enthält feuchtigkeitsspendende Mittel vorzugsweise in Mengen von 0,1 bis 10 Gew.-%, bevorzugt von 0,2 bis 8 Gew.-%, insbesondere bevorzugt 0,3 bis 6 Gew.-%, jeweils bezogen auf das Gesamtgewicht der kosmetischen Zusammensetzung.

Optional enthält die kosmetische Zusammensetzung weitere kosmetisch übliche Additive wie perlglanzgebende Mittel.

Beispiele für vorzugsweise eingesetzte perlglanzgebende Mittel sind Perlglanzpigmente oder Glycol Distearate.

Die erfindungsgemäße kosmetische Zusammensetzung enthält Perlglanz gebende Mittel vorzugsweise in Mengen von Mengen von 0,1 bis 7 Gew.-%, bevorzugt von 0,2 bis 6 Gew.-%, insbesondere bevorzugt 0,3 bis 5 Gew.-%, jeweils bezogen auf das Gesamtgewicht der kosmetischen Zusammensetzung.

Die kosmetische Zusammensetzung wird durch Mischen mindestens einer erfindungsgemäßen wässrigen Dispersion von vorvernetzten Organopolysiloxanen mit mindestens einem Haar-Konditionierungsmittel und ggf. weiteren kosmetisch üblichen Additiven in einem kosmetisch akzeptablen Medium hergestellt.

Die einzelnen Inhaltsstoffe können in einem heiß/heiß-, heiß/kalt- oder kalt/kalt-Verfahren miteinander vermischt werden.

Die Zugabe der erfindungsgemäßen Dispersionen von vorvernetzten Organopolysiloxanen beim Herstellen der erfindungsgemäßen kosmetischen Zusammensetzung erfolgt vorzugsweise bei Temperaturen von höchstens 50°C, bevorzugt bei Temperaturen von höchstens 40°C, besonders bevorzugt bei Temperaturen von höchstens 35°C. Sie erfolgt vorzugsweise bei Temperaturen von mindestens 5°C, bevorzugt bei Temperaturen von mindestens 10°C. Die erfindungsgemäße kosmetische Zusammensetzung kann vorliegen in Form einer Emulsion, einer Suspension, einer Lösung, einer Creme, einer Lotion, eines Schaums, eines Stifts, einer Paste, eines Gels.

Die erfindungsgemäße kosmetische Zusammensetzung in Form einer Emulsion kann vorliegen in Form einer W/O-Emulsion (Wasser-in-Öl-Emulsion), einer O/W-Emulsion (Öl-in-Wasser-Emulsion) oder als multiple Emulsion.

Ist das Ziel, eine kosmetische Zusammensetzung enthaltend erfindungsgemäße wässrige Dispersion von vorvernetzten Organopolysiloxanen in Form einer Emulsion in transluzenter oder transparenter Erscheinungsform herzustellen, werden bevorzugt erfindungsgemäße wässrige Dispersion von vorvernetzten Organopolysiloxanen mit Teilchengrößen < 60 nm eingesetzt, besonders bevorzugt mit Teilchengrößen < 50 nm, insbesondere bevorzugt mit Teilchengrößen < 40 nm.

Vorzugsweise werden die kosmetischen Zusammensetzungen zur Reinigung und Pflege von Haaren verwendet.

Beispiele für Mittel zur Reinigung und Pflege von Haaren sind Haar-Shampoos, -Spülungen (Rinse-Off Conditioner), -Kuren, - Masken, -Seren, -Schäume, -Stylingsprays, -Cremes, -Gele, -Öle, -Spitzenfluids und -Färbemittel.

## Beispiele:

### Beispiele 1-6:

Die nachfolgenden Beispiele 1-6 repräsentieren Herstellverfahren zur Synthese von erfindungsgemäßen wässrigen Dispersionen von vorvernetzten Organopolysiloxanen für erfindungsgemäße kosmetische Zusammensetzungen Die in den Beispielen eingesetzten Aminöle sind Hydroxyterminiert oder gemischt Hydroxy-Methoxy-terminiert.

### Beispiel 1:

Mit Hilfe eines Dissolvers Typ LDV 1 der Firma PC Laborsystem bereitet man aus 6,5 g Isotridecylpentaethoxylat, käuflich erwerblich unter dem Handelsnamen Lutensol TO 5 (Fa. BASF), 20,0 g eines Copolymers aus 3-(2-Aminoethylamino)propyl-methylsiloxy- und Dimethylsiloxyeinheiten mit einer Aminzahl von 0,13 und einer Viskosität von 3900 $mm^2$/s (bei 25°C), 2,9 g Glycerin, 0,12 g 80%iger Essigsäure, 0,19 g N-Morpholinomethyltriethoxysilan und 70 g Wasser eine Mikroemulsion mit einer durchschnittlichen Teilchengröße von 20 bis 50 nm. In diese Emulsion werden 0,13 g Phenoxyethanol, käuflich erwerblich unter dem Handelsnamen S&M Phenoxyethanol (Schülke & Mayr GmbH & CO KG) eingemischt.

### Beispiel 2:

Mit Hilfe eines Dissolvers Typ LDV 1 der Firma PC Laborsystem bereitet man aus 1,5 g Isotridecyldecaethoxylat, käuflich erwerblich unter dem Handelsnamen Lutensol TO 109 (Fa. BASF), 35,0 g eines Copolymers aus 3-(2-Aminoethylamino)propyl-methylsiloxy- und Dimethylsiloxyeinheiten mit einer Aminzahl von 0,16 und einer Viskosität von 4300 $mm^2$/s (bei 25°C), 0,16 g 80%iger Essigsäure, 0,37 g N-Morpholinomethyltriethoxysilan und 60 g Wasser eine Makroemulsion mit einer durchschnittlichen Teilchengröße von 150 bis 280 nm. In diese Emulsion werden 0,13 g Phenoxyethanol, käuflich erwerblich unter dem Handelsnamen S&M Phenoxyethanol (Schülke & Mayr GmbH & CO KG) eingemischt.

### Beispiel 3:

Mit Hilfe eines Dissolvers Typ LDV 1 der Firma PC Laborsystem bereitet man aus 6,0 g Isotridecylpentaethoxylat, käuflich erwerblich unter dem Handelsnamen Lutensol TO 5 (Fa. BASF), 1,5 g Isotridecyldecaethoxylat, käuflich erwerblich unter dem Handelsnamen Lutensol TO 109 (Fa. BASF), 15,3 g eines Copolymers aus 3-(2-Aminoethylamino)propyl-methylsiloxy- und Dimethylsiloxyeinheiten mit einer Aminzahl von 0,26 und einer Viskosität von 1000 $mm^2$/s (bei 25°C), 3,6 g Glycerin, 0,25 g 99%iger Essigsäure, 0,30 g N-Morpholinomethyltriethoxysilan und 74,2 g Wasser eine Mikroemulsion mit einer durchschnittlichen Teilchengröße von 20 bis 60 nm. In diese Emulsion werden 0,9 g Phenoxyethanol, käuflich erwerblich unter dem Handelsnamen S&M Phenoxyethanol (Schülke & Mayr GmbH & CO KG) eingemischt.

Nach Eindampfen der Emulsion über einen Zeitraum von 48h / 25°C verbleibt ein elastisches, gelartiges Material. 3 g des so entstandenen elastischen Materials sind in 7 g Toluol nicht löslich.

### Beispiel 4:

Mit Hilfe eines Dissolvers Typ LDV 1 der Firma PC Laborsystem bereitet man aus 6,1 g Isotridecyldecaethoxylat, käuflich erwerblich unter dem Handelsnamen Lutensol TO 109 (Fa. BASF), 57,6 g eines Polydimethylsiloxandiols mit einem Gehalt an terminalen OH-Gruppen von 1100 Gew.-ppm, 18,4 g PEG-400, käuflich erwerblich unter dem Handelsnamen Polyglykol 400 (Fa. Clariant), 0,41 g N-Morpholinomethyltriethoxysilan und 17,3 g Wasser eine Makroemulsion. In diese Emulsion werden 0,13 g Phenoxyethanol, käuflich erwerblich unter dem Handelsnamen S&M Phenoxyethanol (Schülke & Mayr GmbH & CO KG) eingemischt.

**Beispiel 5:**

Mit Hilfe eines Dissolvers Typ LDV 1 der Firma PC Laborsystem bereitet man aus 6,1 g Isotridecyldecaethoxylat, käuflich erwerblich unter dem Handelsnamen Lutensol TO 109 (Fa. BASF), 29,1 g eines Polydimethylsiloxandiols mit einem Gehalt an terminalen OH-Gruppen von 1100 Gew.-ppm, 28,9 g eines Copolymers aus 3-(2-Aminoethylamino)propyl-methylsiloxy- und Dimethylsiloxyeinheiten mit einer Aminzahl von 0,16 und einer Viskosität von 4300 mm$^2$/s (bei 25°C), 18,4 g PEG-400, käuflich erwerblich unter dem Handelsnamen Polyglykol 400 (Fa. Clariant), 0,43 g N-Morpholinomethyltriethoxysilan und 17,3 g Wasser eine Makroemulsion. In diese Emulsion werden 0,13 g Phenoxyethanol, käuflich erwerblich unter dem Handelsnamen S&M Phenoxyethanol (Schülke & Mayr GmbH & CO KG) eingemischt.

**Beispiel 6:**

Mit Hilfe eines Dissolvers Typ LDV 1 der Firma PC Laborsystem bereitet man aus 1,5 g Isotridecyldecaethoxylat, käuflich erwerblich unter dem Handelsnamen Lutensol TO 109 (Fa. BASF), 35,1 g eines Copolymers aus 3-(2-Aminoethylamino)propyl-methylsiloxy- und Dimethylsiloxyeinheiten mit einer Aminzahl von 0,16 und einer Viskosität von 4300 mm$^2$/s (bei 25°C), 0,16 g 80%iger Essigsäure, 0,12 g N-Morpholinomethyltriethoxysilan, 0,22 g N-Morpholinomethyldiethoxysilan und 60 g Wasser eine Makroemulsion mit einer durchschnittlichen Teilchengröße von 150 bis 280 nm. In diese Emulsion werden 0,13 g Phenoxyethanol, käuflich erwerblich unter dem Handelsnamen S&M Phenoxyethanol (Schülke & Mayr GmbH & CO KG) eingemischt.

**Beispiel 7 :**

Mit Hilfe eines Dissolvers Typ LDV 1 der Firma PC Laborsystem bereitet man aus 3,9 g Isotridecyldecaethoxylat, käuflich erwerblich unter dem Handelsnamen Lutensol TO 109 (Fa. BASF), 35,1 g eines Copolymers aus 3-(2-Aminoethylamino)propyl-methylsiloxy- und Dimethylsiloxyeinheiten mit einer Aminzahl von 0,16 und einer Viskosität von 4300 mm$^2$/s (bei 25°C), 0,16 g 80%iger Essigsäure, 0,37 g [(Cyclohexylamino)methyl]triethoxy-silan und 60 g Wasser eine Makroemulsion mit einer durchschnittlichen Teilchengröße von 160 bis 240 nm. In diese Emulsion werden 0,13 g Phenoxyethanol, käuflich erwerblich unter dem Handelsnamen S&M Phenoxyethanol (Schülke & Mayr GmbH & CO KG) eingemischt.

**Vergleichsversuche V-1 bis V-4:**

Die nachfolgenden Vergleichsversuche V-1 bis V-4 repräsentieren Herstellverfahren zur Synthese wässriger Dispersion von nicht vorvernetzten Organopolysiloxanen für nicht erfindungsgemäße kosmetische Zusammensetzungen.
Die in den Beispielen eingesetzten Aminöle sind Hydroxyterminiert oder gemischt Hydroxy-Methoxy-terminiert.
Es wurde die Arbeitsweise der Beispiele 1-4 im folgendem wiederholt, mit der Abänderung, dass kein N-Morpholinomethyltriethoxysilan zugegeben wird, und daher die Organopolysiloxane nicht vorvernetzt bzw. unvernetzt sind.

**Vergleichsversuch V-1:**

Mit Hilfe eines Dissolvers Typ LDV 1 der Firma PC Laborsystem bereitet man aus 6,5 g Isotridecylpentaethoxylat, käuflich erwerblich unter dem Handelsnamen Lutensol TO 5(Fa. BASF), 20,5 g eines Copolymers aus 3-(2-Aminoethylamino)propyl-methylsiloxy- und Dimethylsiloxyeinheiten mit einer Aminzahl von 0,13 und einer Viskosität von 3900 mm$^2$/s (bei 25°C), 2,9 g Glycerin, 0,11 g 80%iger Essigsäure und 73,0 g Wasser eine Mikroemulsion mit einer durchschnittlichen Teilchengröße von 20 bis 50 nm. In diese Emulsion werden 0,13 g Phenoxyethanol, käuflich erwerblich unter dem Handelsnamen S&M Phenoxyethanol (Schülke & Mayr GmbH & CO KG) eingemischt.

**Vergleichsversuch V-2:**

Mit Hilfe eines Dissolvers Typ LDV 1 der Firma PC Laborsystem bereitet man aus 1,5 g Isotridecyldecaethoxylat, käuflich erwerblich unter dem Handelsnamen Lutensol TO 109(Fa. BASF), 35,0 g eines Copolymers aus 3-(2-Aminoethylamino)propyl-methylsiloxy- und Dimethylsiloxyeinheiten mit einer Aminzahl von 0,16 und einer Viskosität von 4300 $mm^2$/s (bei 25°C), 0,16 g 80%iger Essigsäure und 62,0 g Wasser eine Makroemulsion mit einer durchschnittlichen Teilchengröße von 150 bis 280 nm. In diese Emulsion werden 0,13 g Phenoxyethanol, käuflich erwerblich unter dem Handelsnamen S&M Phenoxyethanol (Schülke & Mayr GmbH & CO KG) eingemischt.

**Vergleichsversuch V-3:**

Mit Hilfe eines Dissolvers Typ LDV 1 der Firma PC Laborsystem bereitet man aus 6,0 g Isotridecylpentaethoxylat, käuflich erwerblich unter dem Handelsnamen Lutensol TO 5 (Fa. BASF), 1,2 g Isotridecyldecaethoxylat, käuflich erwerblich unter dem Handelsnamen Lutensol TO 109 (Fa. BASF), 15,3 g eines Copolymers aus 3-(2-Aminoethylamino)propyl-methylsiloxy- und Dimethylsiloxyeinheiten mit einer Aminzahl von 0,26 und einer Viskosität von 1000 $mm^2$/s (bei 25°C), 3,6 g Glycerin, 0,25 g 99%iger Essigsäure und 76,5 g Wasser eine Mikroemulsion mit einer durchschnittlichen Teilchengröße von 20 bis 60 nm. In diese Emulsion werden 0,9 g Phenoxyethanol, käuflich erwerblich unter dem Handelsnamen S&M Phenoxyethanol (Schülke & Mayr GmbH & CO KG) eingemischt.

Nach Eindampfen der Emulsion über einen Zeitraum von 48h / 25°C verbleibt ein trübes, hochviskoses Öl. Drei g des so entstandenen Öls sind in 7 g Toluol löslich.

**Vergleichsversuch V-4:**

Mit Hilfe eines Dissolvers Typ LDV 1 der Firma PC Laborsystem bereitet man aus 6,1 g Isotridecyldecaethoxylat, käuflich erwerblich unter dem Handelsnamen Lutensol TO 109 (Fa. BASF), 57,6 g eines Polydimethylsiloxandiols mit einem Gehalt an terminalen OH-Gruppen von 1100 Gew.-ppm, 18,4 g PEG-400, käuflich erwerblich unter dem Handelsnamen Polyglykol 400 (Fa. Clariant) und 18,0 g Wasser eine Makroemulsion. In diese Emulsion werden 0,13 g Phenoxyethanol, käuflich erwerblich unter dem Handelsnamen S&M Phenoxyethanol (Schülke & Mayr GmbH & CO KG) eingemischt.

**Testmethoden:**

Testmethoden zur Beurteilung der Wirkung von kosmetischen Zusammensetzungen: Die Beurteilung des Applikationsverhaltens der kosmetischen Zusammensetzung und deren Wirkung bezüglich Benetzung mit Wasser / Hydrophobierung, Kämmkraft, Weichheit und Farbschutz auf keratinischen Fasern erfolgte auf kaukasischem Haar. Alle Haartressen werden vor ihrem Einsatz gereinigt. Hierzu werden die Haartressen 24 Stunden in ein Lösungsmittelgemisch aus gleichen Teilen Aceton, Ethanol, Isopropanol und voll entsalztem Wasser eingelegt. Nach Entfernen des Lösungsmittelgemischs werden die Haartressen gründlich mit voll entsalztem Wasser gewaschen. Anschließend wird jede Haartresse mit 3 ml einer Ammonium Lauryl Sulfat Lösung (25 %), STEPANOL® ALS 25, Fa. STEPAN Company, gewaschen und so lange mit voll entsalztem Wasser gespült, bis kein Schaum mehr sichtbar ist (mindestens 2 Minuten). Nach der Grundreinigung werden die Tressen vor ihrer Weiterverwendung mindestens 12 Stunden bei 23°C und 60 % Luftfeuchte konditioniert.

Benetzung mit Wasser - Hydrophobie von Haaren:

Das Messprinzip zur Bestimmung der Hydrophobie von Haaren folgt den Ausführungen in DE 198 26 081 A1 bzw. DE 10 2010 035 844 Die Bestimmung des Benetzungsverhaltens von keratinischen Fasern mit Wasser erfolgte an Haartressen. Zu diesem Zweck wurden Haartressen aus einer Lieferung von geschädigtem, kaukasischem Haar der Fa. Kerling International Haarfabrik GmbH (Haartressen Schädigungsgrad B, doppelt gezogen) mit einem Gewicht von 2 g und einer Länge von 20 cm verwendet - optionsweise unbehandelt oder behandelt mit einer kosmetischen Zusammensetzung. Die zu untersuchende Haartresse wurde von Richtung Haarwurzel zu Haarspitze in jeweils 2 cm lange Teilstücke unterteilt und derart vorbereitet, dass die Teilstücke der Haartresse mit einem Kunststoffrohr eines Durchmessers von ca. 0,5 cm ummantelt sind. Die Teilstücke enthaltend die Haarwurzel und Haarspitze werden verworfen. Die weiteren präparierten Probenkörper wurden zeitgleich auf einen Probenhalter in ein Becken überführt, das mit deionisiertem, auf 25°C temperiertem Wasser gefüllt ist. Der Probenhalter ist in einer Art konstruiert, die Proben auf gleicher Ebene und senkrecht zum Beckenboden zu positionieren. Ein Aufsteigen der Probenkörper durch Auftriebskräfte ist durch den Probenhalter unterbunden. Ein widerstandsfreies Absinken der Probenkörper auf den Beckenboden ist durch den Probenhalter gewährleistet. Nach Einbringen der Probenkörper in das Wasserbecken erfolgte die Benetzung der Kunststoffrohr ummantelten Haare mit Wasser. Bei ausreichender Benetzung der Haaroberfläche mit Wasser sank der jeweilige Probenkörper vom Probenhalter auf den Boden des Wasserbeckens. Die Zeit bis zum Absinken des Probenkörpers korreliert mit der Hydrophobie der Haaroberfläche und ist länger, je hydrophober die Haaroberfläche ist. Die Zeit bis zum Absinken der Probenkörper in Sekunden wurde erfasst und als Maß für die Hydrophobie der Haaroberfläche korreliert. Pro Messung wurden mindestens drei Haartressen beurteilt. Als Messergebnis wurde der Mittelwert der Probenkörper über alle vermessenen Haartressen verwendet.

Kämmkraftmessung:

Zur Bestimmung der Kämmkraft von nassem und trockenen Haar wurden Haartressen aus geschädigtem, kaukasischem Haar der Fa. Kerling International Haarfabrik GmbH (Haartressen Schädigungsgrad B , doppelt gezogen) mit einem Gewicht von 2 g und einer Länge von 20 cm verwendet.

Die Messung der Kämmkraft mit Doppelkammmethode nach Y.K. Kamath und Hans-Dietrich Weigmann, J. Soc. Cosmet. Chem., 37, 111-124, 1986 erfolgte mit einer Zug-Dehnungsmaschine Instron 3343.

Zunächst wird die Nass- und Trockenkämmkraft entlang der Messstrecke an unbehandelten Haartressen bestimmt. Anschließend werden die Haartressen mit einer erfindungsgemäßen kosmetischen Zusammensetzung behandelt und die Kraftaufnahme beim Kämmvorgang bestimmt.

Als Messwert wird die Reduktion der Kämmkraft entlang der Messstrecke (Arbeit) angegeben, die sich zwischen der behandelten und unbehandelten Haartresse ergibt. Es wird der Mittelwert aus drei Haartressen gebildet. Die Angabe der Kämmkraftreduktion erfolgt in Prozent.

Weichheit:

Das Messprinzip zur Bestimmung der Hydrophobie von Haaren folgt den Ausführungen in DE 10 2010 020 192. Zur Bestimmung der Weichheit des Haars wurden Haartressen aus geschädigtem, kaukasischem Haar der Fa. Kerling International Haarfabrik GmbH (Haartressen Schädigungsgrad B , doppelt gezogen) mit einem Gewicht von 2 g und einer Länge von 20 cm verwendet. Die Bestimmung der Haarweichheit im trockenen Zustand erfolgte durch Einsatz einer Zug-Prüfmaschine Instron 3343, indem die benötigte Zugkraft mit den Parametern Biegesteifigkeit und Oberflächenrauhigkeit des Haarbündels in Korrelation gesetzt wird. Diese beiden Parameter wiederum korrelieren mit der Haarweichheit. Zu diesem Zweck wurde eine unbehandelte Haartresse in eine Messanordnung eingespannt, die aus fünf versetzt gegenüberliegenden Stäben besteht. Die Form der Haartresse in dieser Ausgangsposition ist eine Art Doppel-S. Die Haartresse wird nach dieser Vorbereitung in einer Richtung aus der Messanordnung gezogen und die notwendige Kraft entlang der Messstrecke als Arbeit ausgewertet. Anschließend werden die Haartressen mit einer erfindungsgemäßen kosmetischen Zusammensetzung behandelt und die Kraftaufnahme beim Ziehen der Haartresse durch die Messanordnung entlang der Messstrecke bestimmt. Als Messwert wird die Reduktion der Zugkraft entlang der Messstrecke (Arbeit) angegeben, die sich zwischen der behandelten und unbehandelten Haartresse ergibt. Eine hohe Reduktion der Zugkraft (Arbeit) entspricht einem guten Weichgriff. Es wird der Mittelwert aus drei Haartressen gebildet.

Aufwaschprozedur Shampoo:

Auf eine gereinigte, befeuchtete Haartresse wird 0,1 g Shampoo pro g Haar appliziert. Das Shampoo wird 30 Sekunden lang in Richtung der Haarspitzen einmassiert. Anschließend wird die Haartresse 30 s unter fließendem, voll entsalztem Wasser ausgespült und mit einem grobzinkigen Kamm entwirrt. Die Prozedur wird zweimal wiederholt. Beim letzten Mal wird der Spülprozess auf 60 s verlängert. Anschließend wird die Haartresse mindestens 12 h bei einer Luftfeuchte von 60% und einer Temperatur von 23°C getrocknet.

Aufwaschprozedur Conditioner:

Auf eine gereinigte, befeuchtete Haartresse wird 0,5 g Rinse-Off Conditioner / g Haar appliziert. Der Rinse-Off Conditioner wird 120 Sekunden lang in Richtung der Haarspitzen einmassiert. Anschließend wird die Haartresse 60 s unter fließendem, voll entsalztem Wasser ausgespült und mit einem grobzinkigen Kamm entwirrt. Die Prozedur wird wiederholt. Anschließend wird die Haartresse mindestens 12 h bei einer Luftfeuchte von 60% und einer Temperatur von 23°C getrocknet.

Farbschutz / Farbmessung:

Eine Beurteilung der erfindungsgemäßen kosmetischen Zusammensetzung bezüglich der Farbschutzwirkung auf keratinischen Fasern erfolgte auf gefärbten Echthaar. Hierzu wurden Haartressen mit einem Gewicht von 4 g und einer Länge von 20 cm aus geschädigtem, kaukasischem Haar der Fa. Kerling International Haarfabrik GmbH verwendet (Klebetresse dicht aus Euro-Haar, Bleichstufe A, Mischung 79). Die Haartressen wurden rot gefärbt. Als Farbton wurde Majirouge 6.66 der Firma L'Oreal eingesetzt. 50 ml Haarfarbpaste wurden mit 80 ml einer sechsprozentigen Wasserstoffperoxidlösung vermischt. Die Paste wurde gleichmäßig auf die Haartressen aufgetragen. Nach 40 Minuten Einwirkzeit bei Raumtemperatur wurde die Farbpaste aus den Haartressen ausgewaschen. Nach Behandlung der Haartresse mit einer Tensidlösung (auf 5 % Aktivgehalt verdünntes STEPANOL® ALS 25, Fa. STEPAN Company) und Trocknung der Tressen wurde der Färbevorgang wiederholt.

Die Farbmessung erfolgt an der glatten Oberfläche der Haarbündel mit dem Farbmessgerät Spectro Guide der Firma Byk-Gardner. Die Farbparameter L, a, b (Lab-Farbraum) werden protokolliert.

Beurteilung des Farbschutzes durch eine Haarbehandlung mit erfindungsgemäßen kosmetischen Zusammensetzungen: Gefärbte Haare verändern den Farbeindruck durch Waschen mit Shampoo. Die Änderung des Farbeindrucks nach einem Waschzyklus kann man durch den $\Delta E$-Wert beschreiben, der definiert ist als:

$$\Delta E = ((L1-L0)^2 + (a1-a0)^2 + (b1-b0)^2)^{1/2}$$

L0, a0, b0 sind die Farbwerte eines gefärbten, unbehandelten Haarbündels.

L1, a1 und b1 sind die Farbwerte des Haarbündels nach dem Waschzyklus.

Ein Waschzyklus definiert sich folgendermaßen:

Ein Haarbündel wird behandelt mit einer erfindungsgemäßen kosmetischen Zusammensetzung, gefolgt von zwei Shampoowäschen unter Verwendung eines kommerziellen, siliconfreien Shampoos. Die Behandlung mit einer erfindungsgemäßen kosmetischen Zusammensetzung kann erfolgen in Form eines Leave-in Produkts oder eines Rinse-Off Produkts

Messung des Farbschutzeffekts:

Nach 1,2,3 und 4 Waschzyklen wird jeweils der $\Delta E$-Wert im Vergleich zum Referenzwert ermittelt. Als Kontrolle dient ein Haarbündel, dass zwischen den Shampoowäschen mit einer kosmetischen Zusammensetzung behandelt wird, die sich von der erfindungsgemäßen Zusammensetzung nur dadurch unterscheidet, dass sie keine Dispersion von vorvernetzten Organopolysiloxanen enthält.

Ein größerer $\Delta E$-Wert ist Hinweis für eine größere Farbänderung. Der Farbschutzeffekt kann unter Verwendung der folgenden Formel als Verbesserung des $\Delta E$-Werts ausgedrückt werden:

$$\% \text{ Farbschutz entspricht } \% \Delta E \text{ Verbesserung} = ((\Delta E(\text{behandelt}) - \Delta E(\text{Kontrolle})) / \Delta E(\text{Kontrolle})) \times 100$$

Curl Retention:

Bei dieser Methode zur Ermittlung der Haarfixierung werden die prozentualen Veränderungen von Ausgangs- zu Endlänge von definiert präparierten Locken im Vergleich zur Haartressenlänge aufgezeichnet.

Haarbündel werden aus ungeschädigtem, kaukasischem Haar der Fa. Kerling International Haarfabrik GmbH präpariert. Haartressen mit 15 cm Länge und 3,5 g Gewicht werden jeweils mit einem Bindfaden gebunden und mit einem geeignetem Klebstoff dauerhaft fixiert. Die Haartressen werden mit Shampoo gereinigt und mit voll entsalztem Wasser ausgewaschen. Die Haartressen werden gekämmt, auf einen Kunststoffstab mit einem Durchmesser von 1,1 cm gewickelt, mit einer Hülle temporär fixiert und bei 50 °C über Nacht getrocknet. Die Locken werden von den Kunststoffstäben abgestreift und anschließend mit der erfindungsgemäßen kosmetischen Zusammensetzung behandelt. Nach einer einstündigen Trocknungsphase bei Raumtemperatur werden die Locken an einer skalierten Hängevorrichtung in einen Klimaschrank bei 23 °C und 90% relativer Luftfeuchtigkeit befestigt. Zuvor wurde die Ausganglänge der Locke bestimmt und notiert. In bestimmten Zeitintervallen wird die Lockenlänge als Veränderung zur Ausgangslänge über einen Zeitraum von 24 h abgelesen. Die Curl Retention wird als prozentuale Veränderung gegenüber dem Ausgangszustand angegeben. Eine hoher Wert für die Curl Retention bedeutet einen guten Halt bzw. gute Fixierung.

Im folgenden beziehen sich alle Angaben in Teilen auf Gew.-teile.

### Beispiele 8A bis 8E

Kosmetische Zusammensetzung: Rinse-Off Conditioner (Spülung) Die nachfolgenden Beispiele repräsentieren kosmetische Zusammensetzungen enthaltend - in einem kosmetisch akzeptablen Medium - mindestens ein Haar-Konditionierungsmittel und mindestens eine wässrige Dispersion von vorvernetzten Organopolysiloxanen aus den Beispielen 1 bis 7. Der Aktivgehalt an Organopolysiloxan in der kosmetischen Zusammensetzung beträgt 2 Gew-%.

**Tabelle 1a:** Rinse-Off Conditionerformulierung 8A bis 8E. Angabe in Gew.-teilen.

| Bestandteile (INCI-name) | Bsp. 8A | Bsp. 8B | Bsp. 8C | Bsp. 8D | Bsp. 8E |
|---|---|---|---|---|---|
| Water | ad 100 | ad 100 | ad 100 | ad 100 | ad 100 |
| Hydroxyethylcellulose [1] | 1,2 | 1,2 | 1,2 | 1,2 | 1,2 |
| Cetyl Alcohol [2] | 1,0 | 1,0 | 1,0 | 1,0 | 1,0 |
| Polysorbate 80 [3] | 1,0 | 1,0 | 1,0 | 1,0 | 1,0 |
| Behentrimonium Chloride [4] | 1,8 | 1,8 | 1,8 | 1,8 | 1,8 |
| Stearamidopropyl Dimethylamine [5] | 0,5 | 0,5 | 0,5 | 0,5 | 0,5 |
| Stearyl Alcohol [6] | 3,0 | 3,0 | 3,0 | 3,0 | 3,0 |
| Citric Acid [7] | 0,2 | 0,2 | 0,2 | 0,2 | 0,2 |
| Tetrasodium EDTA [8] | 0,2 | 0,2 | 0,2 | 0,2 | 0,2 |
| Emulsion aus Beispiel 1 | 10,0 | | | | |
| Emulsion aus Beispiel 2 | | 5,7 | | | |
| Emulsion aus Beispiel 3 | | | 13,3 | | |
| Emulsion aus Beispiel 4 | | | | 3,3 | |
| Emulsion aus Beispiel 5 | | | | | 3,0 |
| Methylchloroisothiazolinone und Methylisothiazolinone [9] | 0,1 | 0,1 | 0,1 | 0,1 | 0,1 |

Die Rohstoffe sind unter folgenden Handelsnamen erhältlich:

1) Hydroxyethylcellulose: Tylose® H 4000 P2, Shin-Etsu Chem. Co.

2) Cetylalkohol: Cetylalkohol, Merck KGaA

3) Polysorbate 80: Tween™ 80, Croda GmbH

4) Behentrimonium Chloride: Genamin® KDMP, Clariant GmbH

5) Stearamidopropyl Dimethylamine, Incromine™ SB , Croda GmbH

6) Stearylalkohol: Stearylalkohol Merck KGaA

7) Citric Acid: Citric Acid, Sigma

8) Tetrasodium EDTA: EDETA® B Pulver, BASF Corporation

9) Methylchloroisothiazolinone und Methylisothiazolinone: Kathon™ CG, Rohm and Haas Company, Inc.

Herstellanweisung:

Wasser wird vorgelegt und unter Rühren auf 75°C erwärmt. Dabei werden 1,2 Teile Hydroxyethylcellulose zugegeben. Wenn 65°C erreicht sind, werden 0,5 Teile Stearamidopropyl Dimethylamine, 1 Teil Polysorbate 80, 3 Teile Stearyl Alcohol, 1 Teil Cetyl Alcohol und 1,8 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,2 Teile Citric Acid und 0,2 Teile Tetrasodium EDTA zugegeben. Bei 35°C werden 0,1 Teile Methylchloroisothiazolinone/Methylisothiazolinone zugegeben. Unter weiterem Rühren erfolgt die Zugabe der Emulsion aus den Beispielen. Die Formulierung wird 15 Minuten unter Rühren homogenisiert.

**Vergleichsversuche V-5A bis V-5D:**

Kosmetische Zusammensetzung :Rinse-Off-Conditioner (Spülung) Die nachfolgenden Vergleichsversuche 5A-5D repräsentieren nicht-erfindungsgemäße kosmetische Zusammensetzungen enthaltend - in einem kosmetisch akzeptablen Medium - mindestens ein Konditionierungsmittel und eine wässrige Dispersion eines nicht-vorvernetzten Organopolysiloxans. Der Aktivgehalt an Organopolysiloxan beträgt 2 Gew-%.

Bei der Herstellung der kosmetischen Zusammensetzungen entsprechend der Vergleichsversuche V5A-V5D wurde die Arbeitsweise der Beispiele 8A-8D im folgenden wiederholt, mit der Abänderung, dass anstelle der Emulsionen aus Beispiel 1-4 (Emulsionen von erfindungsgemäßen vorvernetzten Organopolysiloxanen) die Emulsionen der Vergleichsversuche V1-V4 eingesetzt werden. Emulsionen der Vergleichsversuche V1-V4 enthalten zu den Beispielen 1-4 bezüglich Ausgangsviskosität und Aminzahl analoge, aber unvernetzte Organopolysiloxane.

**Tabelle 1b:** Rinse-Off Conditionerformulierung V5A bis V5D. Angabe in Gew.-teilen.

| Bestandteile (INCI-name) | Vergl.-versuch V-5A | Vergl.-versuch V-5B | Vergl.-versuch V-5C | Vergl.-versuch V-5D |
|---|---|---|---|---|
| Water | ad 100 | ad 100 | ad 100 | ad 100 |
| Hydroxyethylcellulose [1] | 1,2 | 1,2 | 1,2 | 1,2 |
| Cetyl Alcohol [2] | 1,0 | 1,0 | 1,0 | 1,0 |
| Polysorbate 80 [3] | 1,0 | 1,0 | 1,0 | 1,0 |
| Behentrimonium Chloride [4] | 1,8 | 1,8 | 1,8 | 1,8 |
| Stearamidopropyl Dimethylamine [5] | 0,5 | 0,5 | 0,5 | 0,5 |
| Stearyl Alcohol [6] | 3,0 | 3,0 | 3,0 | 3,0 |
| Citric Acid [7] | 0,2 | 0,2 | 0,2 | 0,2 |
| Tetrasodium EDTA [8] | 0,2 | 0,2 | 0,2 | 0,2 |
| Emulsion aus Vergleichsversuch V-1 | 10,0 | | | |
| Emulsion aus Vergleichsversuch V-2 | | 5,7 | | |
| Emulsion aus Vergleichsversuch V-3 | | | 13,3 | |
| Emulsion aus Vergleichsversuch V-4 | | | | 3,0 |
| Methylchloroisothiazolinone und Methylisothiazolinone [9] | | 0,1 | 0,1 | 0,1 |

Die Rohstoffe sind unter folgenden Handelsnamen erhältlich:
1) Hydroxyethylcellulose: Tylose® H 4000 P2, Shin-Etsu Chemical Co.
2) Cetylalkohol: Cetylalkohol, Merck KGaA
3) Polysorbate 80: Tween™ 80, Croda GmbH
4) Behentrimonium Chloride: Genamin® KDMP, Clariant GmbH
5) Stearamidopropyl Dimethylamine, Incromine™ SB , Croda GmbH
6) Stearylalkohol: Stearylalkohol Merck KGaA
7) Citric Acid: Citric Acid, Sigma
8) Tetrasodium EDTA: EDETA® B Pulver, BASF Corporation
9) Methylchloroisothiazolinone und Methylisothiazolinone: Kathon™ CG, Rohm and Haas Company, Inc.

Herstellanweisung:

Wasser wird vorgelegt und unter Rühren auf 75°C erwärmt. Dabei werden 1,2 Teile Hydroxyethylcellulose zugegeben. Wenn 65°C erreicht sind, werden 0,5 Teile Stearamidopropyl Dimethylamine, 1 Teil Polysorbate 80, 3 Teile Stearyl Alcohol, 1 Teil Cetyl Alcohol und 1,8 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,2 Teile Citric Acid und 0,2 Teile Tetrasodium EDTA zugegeben. Bei 35°C werden 0,1 Teile Methylchloroisothiazolinone/Methylisothiazolinone zugegeben. Unter weiterem Rühren erfolgt die Zugabe der Emulsion aus dem Vergleichsversuche V-5A bis V-5D. Die Formulierung wird 15 Minuten unter Rühren homogenisiert.

**Vergleich der Rinse-Off Conditioner der erfindungsgemäßen Beispiele 8A-8D mit den Vergleichsversuchen V-5A bis V-5D.**

Die angeführten Beispiele und Vergleichsversuche unterscheiden sich dadurch, dass im Fall der Beispiele 8A-8D wässrige Dispersionen von vorvernetzten Organopolysiloxanen Einsatz fanden, in Vergleichsversuch V-5A bis V-5D jeweils analoge, wässrige Dispersionen der entsprechenden nicht vorvernetzten Organopolysiloxane. Im direkten Vergleich korrelieren folgende Beispiele / Vergleichsversuche:

Beispiel 8A - Vergleichsversuch V-5A
Beispiel 8B - Vergleichsversuch V-5B
Beispiel 8C - Vergleichsversuch V-5C
Beispiel 8D - Vergleichsversuch V-5D

**Hydrophobie, Benetzbarkeit von Haaren**

Ungeschädigtes, natürliches Haar weist eine intakte Lipidschutzschicht an der Haaroberfläche auf und besitzt deshalb eine hohe Hydrophobie. Die Hydrophobie der Haaroberfläche wird mit zunehmenden Schädigungsgrad der Haare geringer, da durch die Zerstörung der Haaroberfläche die wasserabweisende Lipidschutzschicht entlang der Haarfaser beschädigt wird und als Haaroberfläche vermehrt hydrophile, aminosäurebasierte Strukturen wirken. Die Haarhydrophobie kann durch Messung der Zeit bis zum Absinken der Probenkörper entsprechend der Methodenbeschreibung "Benetzung mit Wasser - Hydrophobie von Haaren" quantifiziert werden. Tabelle 2 zeigt die Messwerte für ungeschädigtes und geschädigtes kaukasisches Haar.

Mit Zunahme der Haarschädigung geht typischerweise der Verlust von kosmetischen Eigenschaften wie z.B. Glätte, Weichheit, Glanz oder Farbeindruck einher.

**Tabelle 2:** Ergebnisse der Benetzbarkeit von ungeschädigten (natürlichen) und geschädigten Haartressen / Hydrophobierung durch Korrelation der Haarhydrophobie mit der Zeit bis zum Absinken der Probenkörper entsprechend der Beschreibung "Benetzung mit Wasser - Hydrophobie von Haaren".

|  | Unbehandeltes, ungeschädigtes, kaukasisches Haar[1] | Unbehandeltes, geschädigtes kaukasisches Haar[2] |
|---|---|---|
| **Zeit bis zum Absinken der Probenkörper [s]** | 700 | 1 |
| [1] Fa. Kerling International Haarfabrik GmbH (ungeschädigtes Haar) [2] Fa. Kerling International Haarfabrik GmbH (Haartressen Schädigungsgrad B) | | |

Die Behandlung von geschädigten Haaren mit Rinse-Off Conditionern führt zur Ausbildung eines Schutzfilms auf der Haaroberfläche, der die Hydrophobie im Vergleich zu unbehandeltem, geschädigtem Haar erhöht. Mit Erhöhung der Hydrophobie verbessern sich auch kosmetische Parameter wie z.B. Glätte, Weichheit, Glanz oder Farbeindruck. Die Zunahme der Hydrophobie ist bei den nachfolgend diskutierten Beispielen und Vergleichsversuchen abhängig vom Typ des im Rinse-Off Conditioner eingesetzten Organopolysiloxans. Beispielsweise ist die hydrophobisierende Wirkung von Rinse-Off Conditionern, enthaltend wässrige Dispersionen von aminofunktionalisierten Polydimethylsiloxanen stärker als bei Rinse-Off Conditionern, enthaltend wässrige Dispersionen von Polydimethylsiloxanen. In allen Fällen führt der Einsatz von wässrigen Dispersionen vorvernetzter Organopolysiloxane (Synthesebeschreibung in Beispiel 1-4) zu einer höheren Hydrophobisierung als beim Einsatz von wässrigen Dispersionen der entsprechenden unvernetzten Organopolysiloxane (Synthesebeschreibung in Vergleichsversuchen 1-4) als Bestandteil der Rinse-Off Conditioner. Eine Übersicht über die einzelnen Versuchsergebnisse zur Quantifizierung der Hydrophobie liefern die Tabellen 3-6.

**Tabelle 3:** Rinse-Off Conditioner / Ergebnisse der Benetzbarkeit der Haartressen / Hydrophobierung nach Behandlung mit Rinse-Off Conditioner durch Korrelation der Haarhydrophobie mit der Zeit bis zum Absinken der Probenkörper entsprechend der Beschreibung "Benetzung mit Wasser - Hydrophobie von Haaren".

| | Beispiel 8A | Vergleichsversuch V-5A |
|---|---|---|
| Zeit bis zum Absinken der Probenkörper [s] | 276 | 227 |

Die Behandlung von Haartressen mit einem Rinse-Off Conditioner, enthaltend eine Mikroemulsion eines vorvernetzten aminofunktionellen Siliconöls mit der Aminzahl 0,13 (Beispiel 8A), führt zu einer höheren Hydrophobie der Haare im Vergleich zur Behandlung mit einem Rinse-Off Conditioner, der eine Mikroemulsion eines analogen, nicht vorvernetzten aminofunktionellen Siliconöls als Bestandteil aufweist (Vergleichsversuch V-5A).

**Tabelle 4:** Rinse-Off Conditioner / Ergebnisse der Benetzbarkeit der Haartressen / Hydrophobierung nach Behandlung mit Rinse-Off Conditioner durch Korrelation der Haarhydrophobie mit der Zeit bis zum Absinken der Probenkörper entsprechend der Beschreibung "Benetzung mit Wasser - Hydrophobie von Haaren".

| | Beispiel 8B | Vergleichsversuch V-5B |
|---|---|---|
| Zeit bis zum Absinken der Probenkörper [s] | 235 | 164 |

Die Behandlung von Haartressen mit einem Rinse-Off Conditioner, enthaltend eine Makroemulsion eines vorvernetzten aminofunktionellen Siliconöls mit der Aminzahl 0,16 (Beispiel 8B), führt zu einer höheren Hydrophobie der Haare im Vergleich zur Behandlung mit einem Rinse-Off Conditioner, der eine Makroemulsion eines analogen, nicht vorvernetzten aminofunktionellen Siliconöls als Bestandteil aufweist (Vergleichsversuch V-5B).

**Tabelle 5:** Rinse-Off Conditioner / Ergebnisse der Benetzbarkeit der Haartressen / Hydrophobierung nach Behandlung mit Rinse-Off Conditioner durch Korrelation der Haarhydrophobie mit der Zeit bis zum Absinken der Probenkörper entsprechend der Beschreibung "Benetzung mit Wasser - Hydrophobie von Haaren".

| | Beispiel 8C | Vergleichsversuch V-5C |
|---|---|---|
| Zeit bis zum Absinken der Probenkörper [s] | 1311 | 783 |

Die Behandlung von Haartressen mit einem Rinse-Off Conditioner, enthaltend eine Mikroemulsion eines vorvernetzten aminofunktionellen Siliconöls mit der Aminzahl 0,26 (Beispiel 8C), führt zu einer höheren Hydrophobie der Haare im Vergleich zur Behandlung mit einem Rinse-Off Conditioner, der eine Mikroemulsion eines analogen, nicht vorvernetzten aminofunktionellen Siliconöls als Bestandteil aufweist (Vergleichsversuch V-5C).

**Tabelle 6:** Rinse-Off Conditioner / Ergebnisse der Benetzbarkeit der Haartressen / Hydrophobierung nach Behandlung mit Rinse-Off Conditioner durch Korrelation der Haarhydrophobie mit der Zeit bis zum Absinken der Probenkörper entsprechend der Beschreibung "Benetzung mit Wasser - Hydrophobie von Haaren".

| | Beispiel 8D | Vergleichsversuch V-5D |
|---|---|---|
| Zeit bis zum Absinken der Probenkörper [s] | 31 | 19 |

Die Behandlung von Haartressen mit einem Rinse-Off Conditioner, enthaltend eine Mikroemulsion eines vorvernetzten Siliconöls (Beispiel 8D), führt zu einer höheren Hydrophobie der Haare im Vergleich zur Behandlung mit einem Rinse-Off Conditioner, der eine Mikroemulsion eines analogen, nicht vorvernetzten Siliconöls als Bestandteil aufweist (Vergleichsversuch V-5D). Beispiel 8D und Vergleichsversuch 5D zeigen deutlich, dass die Hydrophobie der behandelten Haare bei Verwendung von Silikonölen im Rinse-Off Conditioner als aktive Komponente deutlich geringer ist, als bei Verwendung von aminofunktionalisierten Siliconölen (Beispiel 8A-8C).

In allen Fällen kann durch den Einsatz von Rinse-Off Conditionern, enthaltend wässrige Dispersionen vorvernetzter Organopolysiloxane, eine höhere Hydrophobie erzielt werden, als bei Einsatz der wässrigen Dispersionen der analogen, nicht vorvernetzten Organopolysiloxane.

Der Einsatz der wässrigen Dispersionen von vorvernetzten Organopolysiloxane im Rinse-Off Conditioner führt zudem zu einer deutlichen Verbesserung von konditionerenden Eigenschaften wie z.B. der Verringerung der Kämmkräfte in nassen und trockenem Zustand oder einer Erhöhung der Weichheit der Haare. Weiterhin tritt ein positiver Effekt bezüglich der Farbbeständigkeit von gefärbtem Haar gegenüber Shampoowäschen ein.

Die Ergebnisse sind nachfolgend für die Rinse-Off Conditioner der Beispiele 8A-8E aufgeführt.

**Tabelle 7:** Rinse-Off Conditioner / Ergebnisse der Trockenkämmkraft- und Nasskämmkraftreduktion und Verbesserung der Weichheit nach Behandlung mit einem erfindungsgemäßen Rinse-Off Conditioner. Alle Ergebnisse beziehen sich auf den Vergleich zu unbehandelten Haartressen.

| Bsp. | Reduktion Trockenkämmkraft [%] | Reduktion Nasskämmkraft [%] | Verbesserung Weichheit [%] |
|---|---|---|---|
| 8A | 76 | 92 | 54 |
| 8B | 55 | 91 | 40 |
| 8C | 46 | 85 | 46 |
| 8D | 61 | 91 | 33 |
| 8E | 17 | 77 | 22 |

In allen Fällen kann durch Behandlung mit einem Rinse-Off Conditioner enthaltend eine wässrige Emulsion eines vorvernetzten Organopolysiloxans eine signifikante Reduktion der Nasskämmkraft und Trockenkämmkraft der Haartressen gemessen werden, die mit einer deutlichen Verbesserung der Haarweichheit einhergeht.

**Tabelle 8:** Rinse-Off Conditioner / Ergebnisse der Farbschutzmessungen nach Einsatz eines erfindungsgemäßen Rinse-Off Conditioners. Alle Ergebnisse im Vergleich zum Blindwert nach vier Waschzyklen.

| Bsp. | Verbesserung im Vergleich zum Blindwert [%] |
|---|---|
| 8A | 9 |
| 8B | 10 |
| 8C | 10 |
| 8D | 8 |

In allen Fällen kann durch Behandlung mit einem Rinse-Off Conditioner enthaltend eine wässrige Emulsion eines vorvernetzten Organopolysiloxans das Auswaschen der roten Haarfarbe verlangsamt werden. Im Vergleich zur Behandlung der Haartressen mit einem Conditioner, enthaltend keine wässrige Emulsion eines vorvernetzten Organopolysiloxans ergibt sich eine Verbesserung zwischen 8 und 10 Prozent nach vier Waschzyklen. Zur Beurteilung der Kenngrößen und Versuchsdurchführung, siehe Details in der Methodenbeschreibung "Farbschutz / Farbmessung".

Zum Vergleich zeigt Tabelle 9 die Ergebnisse für die Trockenkämmkraft- und Nasskämmkraftreduktion nach Einsatz von nicht erfindungsgemäßen Rinse-Off Conditionern enthaltend wässrigen Emulsionen, nicht vorvernetzter Organopolysiloxane.

**Tabelle 9:** Ergebnisse der Trockenkämmkraft- und Nasskämmkraftreduktion von nicht erfindungsgemäßen Rinse-Off Conditionern. Alle Ergebnisse im Vergleich zu unbehandelten Haartressen.

| Vergleichsversuch | Reduktion Trockenkämmkraft [%] | Reduktion Nasskämmkraft [%] |
|---|---|---|
| V-5A | 75 | 87 |
| V-5B | 51 | 85 |
| V-5C | 42 | 81 |
| V-5D | 5 | 83 |

Tendentiell sind die Verbesserungen für Trocken- und Nasskämmkraft bei Einsatz von Dispersionen vorvernetzter Organopolysiloxane in Rinse-Off Conditionern größer (Tabelle 7) als bei Einsatz der entsprechenden Dispersionen nicht vorvernetzter Organopolysiloxane (Tabelle 9).

**Beispiele 9A / 9B bis Beispiel 13:**

Die nachfolgenden Beispiele repräsentieren kosmetische Zusammensetzungen enthaltend - in einem kosmetisch akzeptablen Medium - mindestens ein Konditionierungsmittel und mindestens eine wässrige Dispersion von vorvernetzten Organopolysiloxanen aus den Beispielen 1 bis 7. Der Aktivgehalt an Organopolysiloxan in der kosmetischen Zusammensetzung beträgt 2 Gew-%.

**Beispiel 9A und 9B:**

Kosmetische Zusammensetzung: 2-Phasen Conditioner

**Tabelle 10:** 2-Phasen Leave-In Conditionerformulierungen 9A und 9B. Angabe in Gew.-teilen.

| Bestandteile (INCI name) | Bsp 9A | Bsp. 9B |
|---|---|---|
| Aqua (Water VES) | ad 100 | ad 100 |
| Dimethicone. [1] | 15,0 | 15,0 |
| Propylene Glycol, Diazolidinyl Urea, Methylparaben, Propylparaben [2] | 1,0 | 1,0 |
| Natriumchlorid [3] | 4,0 | 4,0 |
| Emulsion aus Beispiel 1 | 3,0 | |
| Emulsion aus Beispiel 6 | | 1,7 |
| 1) Dimethicone: Wacker-Belsil® DM 1 plus, Wacker-Chemie AG  2) Propylene Glycol, Diazolidinyl Urea, Methylparaben, Propylparaben: Germaben® II, International Specialty Products  3) 25 prozentige Lösung | | |

Herstellanweisung:

Wasser wird vorgelegt. Es wird auf 35 °C erwärmt, dann erfolgt unter Rühren die Zugabe von Natriumchlorid. Nach vollständigem Lösen von Natriumchlorid wird Propylene Glycol, Diazolidinyl Urea, Methylparaben, Propylparaben zugegeben, auf Raumtemperatur abgekühlt, und unter Rühren die Emulsion aus den Beispielen beigemengt. Abschließend wird die Mischung mit Dimethicone vermengt. Man erhält ein Zwei-Phasen-System, dass unter kräftigem Schütteln vermischt werden kann und sich nach einiger Zeit wieder in zwei Phasen trennt.
Die 2-Phasen Conditioner-behandelten Haare wurden in einem Paneltest als sehr weich, seidig und leicht kämmbar bewertet.

**Beispiel 10:**

Kosmetische Zusammensetzung: **Shampoo**

**Tabelle 11:** Shampooformulierungen 10A-10D. Angabe in Gew.-teilen.

| Bestandteile (INCI-name) | Bsp. 10A | Bsp. 10B | Bsp. 10C | Bsp. 10D |
|---|---|---|---|---|
| Aqua (Water VES) | ad 100 | ad 100 | ad 100 | ad 100 |
| Guar Hydroxypropyltrimonium Chloride [1] | 0,20 | 0,20 | 0,20 | 0,20 |
| Sodium Laureth Sulfate [2] | 41,50 | 41,50 | 41,50 | 41,50 |
| Glycol Distearate [3] | 1,20 | 1,20 | 1,20 | 1,20 |
| PEG-150 Distearate [4] | 0,20 | 0,20 | 0,20 | 0,20 |

---

(fortgesetzt)

| Bestandteile (INCI-name) | Bsp. 10A | Bsp. 10B | Bsp. 10C | Bsp. 10D |
|---|---|---|---|---|
| Emulsion aus Beispiel 1 | 10,0 | | | |
| Emulsion aus Beispiel 2 | | 5,7 | | |
| Emulsion aus Beispiel 3 | | | 13,3 | |
| Emulsion aus Beispiel 4 | | | | 3,3 |
| Cocamidopropyl Betaine [5] | 13,42 | 13,42 | 13,42 | 13,42 |
| Methylchloroisothiazolinone und Methylisothiazolinone [6] | 0,06 | 0,06 | 0,06 | 0,06 |
| Sodium Chloride [7] | 0,45 | 0,45 | 0,45 | 0,45 |

1) Guar Hydroxypropyltrimonium Chloride: N-Hance® 3000, Hercules Inc.
2) Sodium Laureth Sulfat: Genapol® LRO 26,5%, Clariant GmbH
3) Glycol Distearate: Genapol® PMS, Clariant GmbH
4) PEG-150 Distearate: Emulgin® EO 33, Cognis Deutschland GmbH
5) Cocamidopropyl Betaine: Genagen® CAB 30%, Clariant GmbH
6) Methylchloroisothiazolinone und Methylisothiazolinone: Kathon™ CG, Rohm and Haas Company, Inc
7) 25-prozentige Lösung

Herstellanweisung:

0,2 Teile Guar Hydroxypropyltrimonium Chloride werden in Wasser dispergiert. 41,5 Teile Sodium Laureth Sulfate werden langsam eingerührt und die Mischung wird schrittweise auf 75°C erwärmt. Während des Erwärmens werden bei 50°C 0,2 Teile PEG-150 Distearate zugegeben, bei 65°C erfolgt die Zugabe von 1,2 Teilen Glycol Distearate.
Anschließend wird die Mischung abgekühlt. Bei Erreichen von 35°C werden 0,06 Teile Methylchloroisothiazolinone/ Methylisothiazolinone und die Emulsion entsprechend der Beispiele zugegeben und 5 Minuten gerührt. Abschließend werden 13,4 Teile Cocamidopropyl Betaine und 0,5 Teile Natriumchlorid zugegeben und jeweils 10 Minuten gerührt.

**Tabelle 12:** Shampoo / Ergebnisse der Trockenkämmkraft- und Nasskämmkraftreduktion. Verbesserung der Weichheit. Alle Ergebnisse im Vergleich zu unbehandelten Haartressen.

| Bsp. | Reduktion Trockenkämmkraft [%] | Reduktion Nasskämmkraft [%] | Verbesserung Weichheit [%] |
|---|---|---|---|
| **10A** | 51 | 83 | 50 |
| **10B** | 64 | 57 | 36 |
| **10C** | 60 | 31 | 23 |
| **10D** | 27 | 51 | 20 |

**Beispiel 11:**

Kosmetische Zusammensetzung: **Shampoo**

**Tabelle 13:** Shampoo Formulierungen 11A und 11B. Angabe in Gew.-teilen.

| Bestandteile (INCI-name) | Bsp. 11A | Bsp. 11B |
|---|---|---|
| Aqua (Water VES) | ad 100 | ad 100 |
| Polyquaternium-10 [1] | 0,10 | 0,10 |
| Sodium Laureth Sulfate [2] | 71,60 | 67,9 |
| Glycol Distearate [3] | 1,20 | 1,20 |

(fortgesetzt)

| Bestandteile (INCI-name) | Bsp. 11A | Bsp. 11B |
|---|---|---|
| Hydroxyethylcellulose [4] | 0,20 | 0,20 |
| Emulsion aus Beispiel 1 | 10,0 | |
| Emulsion aus Beispiel 7 | | 13,3 |
| Cocamidopropyl Betaine [5] | 10,0 | 8,3 |
| Methylchloroisothiazolinone und Methylisothiazolinone [6] | 0,06 | 0,06 |
| Sodium Chloride [7] | 0,5 | 1,0 |

1) Polyquaternium-10: Ucare™ Polymer JR-400, Amerchol Corporation
2) Sodium Laureth Sulfat: Genapol® LRO 26,5%, Clariant GmbH
3) Glycol Distearate: Genapol® PMS, Clariant GmbH
4) Hydroxyethylcellulose: Tylose® H 4000 P2, Shin-Etsu Chemical Co
5) Cocamidopropyl Betaine: Genagen® CAB 30%, Clariant GmbH
6) Methylchloroisothiazolinone und Methylisothiazolinone: Kathon™ CG, Rohm and Haas Company, Inc
7) 25-prozentige Lösung

Herstellanweisung:

0,1 Teile Polyquaternium-10 werden in Wasser dispergiert. Sodium Laureth Sulfate wird langsam eingerührt und die Mischung wird auf 75°C erwärmt. Bei Erreichen von 50°C werden 0,2 Teile Hydroxyethylcellulose zugegeben, bei 65°C werden 1,2 Teile Glycol Distearate zugegeben. Die Mischung wird gerührt, bis 75°C erreicht sind. Dann wird die Mischung abgekühlt. Bei Erreichen von 35°C werden 0,06 Teile Methylchloroisothiazolinone/Methylisothiazolinone und die Emulsion entsprechend der Beispiele zugegeben und 5 Minuten gerührt. Abschließend wird Cocamidopropyl Betaine und Natriumchloridlösung zugegeben und jeweils 10 Minuten gerührt.

**Tabelle 14:** Shampoo / Ergebnis der Trockenkämmkraft- und Nasskämmkraftreduktion. Verbesserung der Weichheit. Alle Ergebnisse im Vergleich zu unbehandelten Haartressen.

| Bsp. | Reduktion Trockenkämmkraft [%] | Reduktion Nasskämmkraft [%] | Verbesserung Weichheit [%] |
|---|---|---|---|
| 11A | 55 | 75 | 52 |
| 11B | 25 | 48 | 38 |

**Beispiel 12:**

Kosmetische Zusammensetzung: Shampoo

**Tabelle 15:** Shampoo Formulierung 12. Angabe in Gew.-teilen.

| Bestandteile (INCI-name) | Bsp. 12 |
|---|---|
| Water | ad 100 |
| Guar Hydroxypropyltrimonium Chloride [1] | 0,2 |
| Polyquaternium-10 [2] | 0,1 |
| Cetearyl Alcohol [3] | 0,5 |
| Glycol Distearate [4] | 1,0 |
| Sodium Laureth Sulfate [5] | 20,0 |
| Cocamidopropyl Betaine [6] | 6,7 |

(fortgesetzt)

| Bestandteile (INCI-name) | Bsp. 12 |
|---|---|
| Cocamide MEA [7] | 0,5 |
| Carbomer [3] | 0,6 |
| Emulsion aus Beispiel 1 | 2,5 |
| Methylchloroisothiazolinone und Methylisothiazolinone [9] | 0,1 |
| Triethanolamin [10] | 1,1 |
| Natrium Chloride [11] | 0,3 |

1) Guar Hydroxypropyltrimonium Chloride: N-Hance® 3000, Hercules Inc.
2) Polyquaternium-10: Ucare™ Polymer JR-400, Amerchol Corporation
3) Cetearyl Alcohol: Crodacol® CS50, Croda Chemicals, Europe Ltd.
4) Glycol Distearate: Genapol® PMS, Clariant GmbH
5) Sodium Laureth Sulfat: Genapol® LRO 26,5%, Clariant GmbH
6) Cocamidopropyl Betaine: Genagen® CAB 30%, Clariant GmbH
7) Cocamide MEA: COMPERLAN® 100, Cognis Coorporation
8) Carbomer: Carbopol 940®, Noveon
9) Methylchloroisothiazolinone und Methylisothiazolinone: Kathon™ CG, Rohm and Haas Company, Inc
10) Triethanolamine: Triethanolamine, Merck KGaA
11) Sodium Chloride: Natrium Chloride, Merck KGaA

Herstellanweisung:

0,2 Teile Guar Hydroxypropyltrimonium Chloride werden in Wasser dispergiert. Unter Erwärmen auf 75°C werden 0,1 Teile Polyquaternium-10, 0,5 Teile Cetearyl Alcohol und 1,0 Teile Glycol Distearate zugegeben. Bei Erreichen von 75°C werden 20 Teile Sodium Laureth Sulfat, 6,7 Teile Cocamidopropyl Betaine und 0,5 Teile Cocamide MEA langsam eingerührt. Carbomer wird in etwas Wasser vorgelöst und zugegeben. Danach wird abgekühlt. Bei Erreichen von 50 °C werden 0,1 Teile Methylchloroisothiazolinone und Methylisothiazolinone zugegeben. Bei 35°C werden 2,5 Teile der Emulsion aus Beispiel 1 und 0,3 Teile Sodium Chloride zugegeben. Anschließend werden 1,1 Teile Triethanolamin zugegeben.

**Tabelle 16:** Shampoo / Ergebnis der Trockenkämmkraft- und Nasskämmkraftreduktion. Verbesserung der Weichheit. Alle Ergebnisse im Vergleich zu unbehandelten Haartressen.

| Bsp. | Reduktion Trockenkämmkraft [%] | Reduktion Nasskämmkraft [%] | Verbesserung Weichheit [%] |
|---|---|---|---|
| **12** | 30 | 18 | 3 |

**Beispiel 13:**

Kosmetische Zusammensetzung: **Haarmousse**

**Tabelle 17:** Haarmousse-Formulierungen 13A - 13C. Angabe in Gew.-teilen.

| Bestandteile (INCI name) | Bsp. 13A | Bsp. 13B | Bsp. 13C |
|---|---|---|---|
| Water | ad 100 | ad 100 | ad 100 |
| PEG-40 Hydrogenated Castor Oil [1] | 0,5 | 0,5 | 0,5 |
| Aminomethyl Propanol [2] | 1,3 | 1,3 | 1,3 |
| Crotonic Acid/Vinyl C8-12 Isoalkyl Esters/VA/Bis-Vinyldimethicone Crosspolymer [3] | 4,0 | 4,0 | 4,0 |

(fortgesetzt)

| Bestandteile (INCI name) | Bsp. 13A | Bsp. 13B | Bsp. 13C |
|---|---|---|---|
| Polyquaternium-11 [4] | 5,0 | 5,0 | 5,0 |
| Cocamidopropyl Betaine [5] | 5,5 | 5,5 | 5,5 |
| Emulsion aus Beispiel 1 | 0,5 | | |
| Emulsion aus Beispiel 4 | | 0,2 | |
| Emulsion aus Beispiel 5 | | | 0,2 |
| Phenoxyethanol and Methylparaben and Ethylparaben and Butylparaben and Propylparaben and Isobutylparaben [6] | 0,5 | 0,5 | 0,5 |
| Propane / Butane | 8,0 | 8,0 | 8,0 |

1) PEG-40 Hydrogenated Castor Oil: Cremophor® RH 40, BASF AG
2) Aminomethyl Propanol, AMP 30%ig
3) SLM 28073, Wacker Chemie AG
4) Polyquaternium-4: Luviquat PQ 11, BASF AG
5) Cocamidopropyl Betaine: Genagen® CAB 30%, Clariant GmbH
6) Phenoxyethanol and Methylparaben and Ethylparaben and Butylparaben and Propylparaben and Isobutylparaben: Phenonip™, Clariant GmbH

Herstellanweisung:

Aminomethyl Propanol wird zusammen mit PEG-40 Hydrogenated Castor Oil unter Rühren in Wasser in einem Becherglas gelöst. Anschließend wird unter weiterem Rühren wird das Styling Polymer Crotonic Acid/Vinyl C8-12 Isoalkyl Esters/VA/Bis-Vinyldimethicone Crosspolymer portionsweise zugegeben und gelöst.

In einem zweiten Becherglas wird Wasser vorgelegt und Polyquaternium-11, Cocamidopropyl Betaine und die Emulsion entsprechend der Beispiele unter Rühren zugegeben. Abschließend erfolgt die Zugabe des Konservierungsmittels. Nun wird die Lösung aus Becherglas 2 zur Lösung aus Becherglas 1 gegeben und homogenisiert. Die Mischung wird in Dosen abgefüllt und mit Propane/Butane als Treibgasgemisch bestückt.

Die mit Styling Mousse behandelten Haare besitzen als Ergebnis der Verwendung einer Kombination eines Silicon-Copolymers als Styling Polymer und der erfindungsgemäßen Dispersion vorvernetzter Organopolysiloxane einen angenehmen Weichgriff.

**Tabelle 18:** Haarmousse / Ergebnis der Curl Retention nach 8 h nach Behandlung mit dem Haarmousse.

| Bsp. | Curl Retention nach 8 h [%] |
|---|---|
| 13A | 28 |
| 13B | 32 |
| 13C | 31 |

**Patentansprüche**

1. Verwendung einer kosmetischen Zusammensetzung enthaltend, in einem kosmetisch akzeptablen Medium, mindestens ein Haar-Konditionierungsmittel ausgewählt aus der Gruppe von
kationischen Polymeren,
kationischen Tensiden,
nicht polymer vorliegenden quaternären Ammoniumverbindungen,
Organopolysiloxanen und Organopolysiloxancopolymeren, die von den vorvernetzten Organopolysiloxanen der Formel (I) verschieden sind,
Fettsäureestern und Fettsäurealkoholen,
natürlichen oder synthetischen Ölen und Wachsen und Panthenol, Lipiden, Proteinen und hydrolysierten Proteinen,
sowie deren Mischungen

und

mindestens eine wässrige Dispersion von vorvernetzten Organopolysiloxanen aus Einheiten der allgemeinen Formel

$$AKT_nR^2{}_bR_c(OR^1)_dSiO_{\frac{4-(n+b+c+d)}{2}} \qquad (I)$$

wobei

R gleich oder verschieden sein kann, einen einwertigen, gesättigten oder ungesättigten Kohlenwasserstoffrest mit 1 bis 200 Kohlenstoffatomen je Rest, der ein oder mehrere Heteroatome aus der Gruppe N, P, S, O, und Halogen enthalten kann, bedeutet,

$R^1$ gleich oder verschieden sein kann, ein Wasserstoffatom oder einen Alkylrest mit 1 bis 8 Kohlenstoffatomen je Rest bedeutet,

$R^2$ gleich oder verschieden sein kann, einen einwertigen, gegebenenfalls substituierten Kohlenwasserstoffrest mit 1 bis 18 Kohlenstoffatomen je Rest bedeutet,

AKT ein Rest der Formel $-CH_2NHR^4$, $-CH_2NR^4{}_2$ oder $-CH_2RN^5$ ist, wobei

$R^4$ einen einwertigen Kohlenwasserstoffrest mit 1 bis 18 Kohlenstoffatomen, der ein oder mehrere Heteroatome aus der Gruppe N und 0 enthalten kann, bedeutet und

$R^5$ einen zweiwertigen Kohlenwasserstoffrest mit 3 bis 12 Kohlenstoffatomen, der ein oder mehrere Heteroatome aus der Gruppe N und O enthalten kann, bedeutet,

b 0, 1 oder 2,

c 0, 1, 2 oder 3,

d 0, 1 oder 2 und

n 0, 1 oder 2 ist,

mit der Maßgabe, dass die Summe $n+b+c+d \leq 3$ ist und dass durchschnittlich je Molekül mindestens ein Rest AKT enthalten ist,

zur Behandlung von keratinischen Fasern, bevorzugt Haaren.

2. Verwendung nach Anspruch 1, **dadurch gekennzeichnet, dass** als wässrige Dispersion eine wässrige Dispersion von vorvernetzten Organopolysiloxanen aus Einheiten der allgemeinen Formel

$$AKT_nR^2{}_bA_mR'{}_c(OR^1)_dSiO_{\frac{4-(n+m+b+c+d)}{2}} \qquad (I') \quad ,$$

enthalten ist, wobei AKT, $R^1$, $R^2$, b, c, d und n die im Anspruch 1 dafür angegebene Bedeutung haben,

R' gleich oder verschieden sind kann, ein einwertiger Kohlenwasserstoffrest mit 1 bis 18 Kohlenstoffatomen bedeutet,

A ein Aminorest der allgemeinen Formel

$$-R^6-[NR^7-R^8-]_zNR^7{}_2,$$

ist, wobei

$R^6$ ein zweiwertiger linearer oder verzweigter Kohlenwasserstoffrest mit 3 bis 18 Kohlenstoffatomen bedeutet,

$R^7$ ein Wasserstoffatom, einen Alkylrest mit 1 bis 8 Kohlenstoffatomen oder einen Acylrest bedeutet,

$R^8$ ein zweiwertiger Kohlenwasserstoffrest mit 1 bis 6 Kohlenstoffatomen bedeutet,

z 0, 1, 2, 3 oder 4 ist, und

m 0 oder 1 ist,

mit der Maßgabe, dass die Summe $n+m+b+c+d \leq 3$ ist und n und m in derselben Siloxaneinheit nicht gleichzeitig 1 sind und dass durchschnittlich je Molekül mindestens ein Rest AKT und ein Rest A enthalten ist.

3. Verwendung nach Anspruch 1 oder 2, **dadurch gekennzeichnet, dass** die wässrige Dispersion von vorvernetzten Organopolysiloxanen hergestellt wird, indem mindestens ein Organopolysiloxan (1) aus Einheiten der allgemeinen

Formel

$$R_c(OR^1)_d SiO_{\frac{4-(c+d)}{2}} \qquad (II)$$

wobei R und $R^1$ die im Anspruch 1 dafür angegebene Bedeutung haben,
c 0, 1, 2 oder 3 und d 0, 1 oder 2 ist,
mit der Maßgabe, dass die Summe $c+d \leq 3$ ist und
im Organopolysiloxan (1) durchschnittlich mindestens ein Rest $OR^1$ je Molekül, bevorzugt in der Bedeutung von $R^1$ gleich Wasserstoffatom, enthalten ist,
mit mindestens einem hochreaktiven Silan (2) der allgemeinen Formel

$$(AKT)_a R^2_b Si(OR^3)_{4-(a+b)} \qquad (III)$$

oder deren Teilhydrolysate

wobei AKT und $R^2$ die im Anspruch 1 dafür angegebene Bedeutung haben,

$R^3$ gleich oder verschieden sein kann, einen Alkylrest mit 1 bis 8 Kohlenstoffatomen je Rest bedeutet,
a 1 oder 2, bevorzugt 1, ist und
b 0, 1 oder 2, bevorzugt 0 oder 1 ist,

mit der Maßgabe, dass die Summe $a+b \leq 3$ ist,
in Gegenwart von Wasser (3),
und Emulgator (4)
umgesetzt wird,
mit der Maßgabe, dass keine metallhaltigen Katalysatoren mit verwendet werden, und
dass Organopolysiloxan (1) und Silan (2) in Art und Menge so gewählt werden, dass die Organopolysiloxane in den erhaltenen Dispersionen vorvernetzt sind.

4. Verwendung nach Anspruch 3, **dadurch gekennzeichnet, dass** die so hergestellten vorvernetzten Organopolysiloxanen nach dem Entfernen des Wassers elastomere Filme bilden.

5. Verwendung nach Anspruch 3 oder 4, **dadurch gekennzeichnet, dass** bei der Herstellung der wässrigen Dispersion von vorvernetzten Organopolysiloxanen als Organopolysiloxane (1) solche der allgemeinen Formel

$$(R^1O)R'_2 SiO(SiAR'O)_p (SiR'_2O)_q SiR'_2(OR^1) \qquad (IVc),$$

eingesetzt werden, wobei

R' gleich oder verschieden sein kann, ein einwertiger Kohlenwasserstoffrest mit 1 bis 18 Kohlenstoffatomen bedeutet,
$R^1$ gleich oder verschieden sein kann, ein Wasserstoffatom oder einen Alkylrest mit 1 bis 8 Kohlenstoffatomen je Rest bedeutet,

der Aminorest **A** die im Anspruch 3 dafür angegebene Bedeutung hat,
p eine ganze Zahl von 1 bis 1000, vorzugsweise 1 bis 10 und q 0 oder eine ganze Zahl von 1 bis 2000, vorzugsweise 50 bis 1000, ist,
mit der Maßgabe, dass 10 bis 100 %, vorzugsweise 20 bis 100 % aller Reste $R^1$ Wasserstoffatome sind.

6. Verwendung nach Anspruch 3, 4 oder 5, **dadurch gekennzeichnet, dass** bei der Herstellung der wässrigen Dispersion von vorvernetzten Organopolysiloxanen als Silan (2) mindestens ein trifunktionelles Silan eingesetzt wird, bei dem a = 1 und b = 0 ist.

7. Verwendung nach einem der Ansprüche 3 bis 6, **dadurch gekennzeichnet, dass** bei der Herstellung der wässrigen Dispersion von vorvernetzten Organopolysiloxanen der Rest - $OR^3$ im Silan (2) ein Ethoxyrest ist.

8. Verwendung nach einem der Ansprüche 1 bis 7, **dadurch gekennzeichnet, dass** der Rest AKT ein Cyclohexylaminomethyl- oder ein Morpholinomethylrest ist.

9. Verwendung nach einem der Ansprüche 1 bis 8, **dadurch gekennzeichnet, dass** als Haar-Konditionierungsmittel solches ausgewählt aus der Gruppe bestehend aus quaternärem Ammoniumderivat eines mit Propylenglycolether modifizierten Cyamopsis Tetragonoloba (Guar) Gums, polymerem quaternären Ammoniumsalz des Reaktionsprodukts von Hydroxyethylcellulose mit Trimethylammoniumsubstituierten Epoxid, Chlorid, Bromid und Methosulfat-Salz des Cetyltrimethylammoniums und Behenyltrimethylammoniums, N-[3-(Dimethylamino)propyl]octadecanamid, 1-Octadecanol und 1-Hexadecanol, sowie deren Mischungen eingesetzt wird.

10. Verwendung nach einem der Ansprüche 1 bis 9, **dadurch gekennzeichnet, dass** das kosmetisch akzeptable Medium Wasser ist.

11. Verwendung nach einem der Ansprüche 1 bis 10, **dadurch gekennzeichnet, dass** die kosmetische Zusammensetzung weitere kosmetisch übliche Additive, ausgewählt aus der Gruppe von
Tensiden, wie anionische, nichtionische und amphotere Tenside,
Verdickern, Gelierungsmitteln,
Filmbildner,
feuchtigkeitsspendenden Mitteln,
UV-Filter,
perlglanzgebenden Mitteln,
Vitaminen,
Antioxidantien,
Coffein,
Anti-Schuppenwirkstoffen und
Konservierungsmitteln,
enthält.

12. Verwendung nach einem der Ansprüche 1 bis 11, **dadurch gekennzeichnet**, das die kosmetische Zusammensetzung durch Mischen mindestens einer wässrigen Dispersion von vorvernetzten Organopolysiloxanen nach einem der Ansprüche 1, 2 oder 8 bis 11 oder hergestellt nach einem der Ansprüche 3 bis 7 mit mindestens einem Konditionierungsmittel
und ggf. weiteren kosmetisch üblichen Additiven in einem kosmetisch akzeptablen Medium hergestellt wird.

**Claims**

1. Use of a cosmetic composition comprising,
   in a cosmetically acceptable medium,
   at least one hair conditioning agent selected from the group of
   cationic polymers,
   cationic surfactants,
   quaternary ammonium compounds present in nonpolymeric form,
   organopolysiloxanes and organopolysiloxane copolymers different from the precrosslinked organopolysiloxanes of the formula (I),
   fatty acid esters and fatty acid alcohols,
   natural or synthetic oils and waxes and
   panthenol, lipids, proteins and hydrolyzed proteins,
   and mixtures thereof
   and
   at least one aqueous dispersion of precrosslinked organopolysiloxanes of units of the general formula

$$AKT_n R^2{}_b R_c (OR^1)_d SiO_{\frac{4-(n+b+c+d)}{2}} \qquad (I)$$

where

R may be identical or different and is a monovalent, saturated or unsaturated hydrocarbon radical having 1 to 200 carbon atoms per radical, which can contain one or more heteroatoms from the group N, P, S, O, and halogen, $R^1$ may be identical or different and is a hydrogen atom or an alkyl radical having 1 to 8 carbon atoms per radical, $R^2$ may be identical or different and is a monovalent, optionally substituted hydrocarbon radical having 1 to 18 carbon atoms per radical,

AKT is a radical of the formula $-CH_2NHR^4$, $-CH_2NR^4_2$ or $-CH_2RN^5$, where

$R^4$ is a monovalent hydrocarbon radical having 1 to 18 carbon atoms, which can contain one or more heteroatoms from the group N and O, and

$R^5$ is a divalent hydrocarbon radical having 3 to 12 carbon atoms, which can contain one or more heteroatoms from the group N and O,

b is 0, 1 or 2,

c is 0, 1, 2 or 3,

d is 0, 1 or 2, and

n is 0, 1 or 2,

with the proviso that the sum n+b+c+d is $\leq 3$ and that on average at least one radical AKT is present per molecule, for treating keratin fibers, preferably hair.

**2.** Use according to Claim 1, **characterized in that** the aqueous dispersion present is an aqueous dispersion of precrosslinked organopolysiloxanes of units of the general formula

$$AKT_n R^2_b A_m R'_c (OR^1)_d SiO_{\frac{4-(n+m+b+c+d)}{2}} \quad (I'),$$

where AKT, $R^1$, $R^2$, b, c, d and n have the meaning given for them in Claim 1,

R' may be identical or different and is a monovalent hydrocarbon radical having 1 to 18 carbon atoms,

A is an amino radical of the general formula

$$-R^6-[NR^7-R^8-]_z NR^7_2,$$

where

$R^6$ is a divalent linear or branched hydrocarbon radical having 3 to 18 carbon atoms,

$R^7$ is a hydrogen atom, an alkyl radical having 1 to 8 carbon atoms or an acyl radical,

$R^8$ is a divalent hydrocarbon radical having 1 to 6 carbon atoms,

z is 0, 1, 2, 3 or 4, and

m is 0 or 1,

with the proviso that the sum n+m+b+c+d is $\leq 3$, and n and m in the same siloxane unit are not simultaneously 1 and that on average at least one radical AKT and a radical A is present per molecule.

**3.** Use according to Claim 1 or 2, **characterized in that** the aqueous dispersion of precrosslinked organopolysiloxanes is prepared by reacting at least one organopolysiloxane (1) of units of the general formula

$$R_c (OR^1)_d SiO_{\frac{4-(c+d)}{2}} \quad (II)$$

where R and $R^1$ have the meaning given for them in Claim 1, c is 0, 1, 2 or 3 and d is 0, 1 or 2, with the proviso that the sum c+d is $\leq 3$ and

in the organopolysiloxane (1) on average at least one radical $OR^1$ is present per molecule, preferably in the meaning

of $R^1$ is hydrogen atom,
with at least one highly reactive silane (2) of the general formula

$$(AKT)_a R^2_b Si(OR^3)_{4-(a+b)} \qquad (III)$$

or part hydrolyzates thereof
where AKT and $R^2$ have the meaning given for them in Claim 1,

$R^3$ may be identical or different and is an alkyl radical having 1 to 8 carbon atoms per radical,
a is 1 or 2, preferably 1, and
b is 0, 1 or 2, preferably 0 or 1,

with the proviso that the sum a+b is $\leq 3$,
in the presence of water (3),
and emulsifier (4),
with the proviso that no metal-containing catalysts are co-used, and
that organopolysiloxane (1) and silane (2) are selected in type and amount such that the organopolysiloxanes are precrosslinked in the resulting dispersions.

4. Use according to Claim 3, **characterized in that** the precrosslinked organopolysiloxanes produced in this way form elastomeric films after removing the water.

5. Use according to Claim 3 or 4, **characterized in that** during the preparation of the aqueous dispersion of precrosslinked organopolysiloxanes, the organopolysiloxanes (1) used are those of the general formula

$$(R^1O)R'_2SiO(SiAR'O)_p(SiR'_2O)_qSiR'_2(OR^1) \qquad (IVc),$$

where

R' may be identical or different and is a monovalent hydrocarbon radical having 1 to 18 carbon atoms,
$R^1$ may be identical or different and is a hydrogen atom or an alkyl radical having 1 to 8 carbon atoms per radical,

the amino radical A has the meaning given for it in Claim 3,
p is an integer from 1 to 1000, preferably 1 to 10 and
q is 0 or an integer from 1 to 2000, preferably 50 to 1000,
with the proviso that 10 to 100%, preferably 20 to 100%, of all radicals $R^1$ are hydrogen atoms.

6. Use according to Claim 3, 4 or 5, **characterized in that** during the preparation of the aqueous dispersion of precrosslinked organopolysiloxanes, the silane (2) used is at least a trifunctional silane, in which a = 1 and b = 0.

7. Use according to any one of Claims 3 to 6, **characterized in that** during the preparation of the aqueous dispersion of precrosslinked organopolysiloxanes, the radical -$OR^3$ in the silane (2) is an ethoxy radical.

8. Use according to any one of Claims 1 to 7, **characterized in that** the radical AKT is a cyclohexylaminomethyl radical or a morpholinomethyl radical.

9. Use according to any one of Claims 1 to 8,
**characterized in that** the hair conditioning agent used is one selected from the group consisting of quaternary ammonium derivative of a cyamopsis tetragonoloba (guar) gum modified with propylene glycol ether,
polymeric quaternary ammonium salt of the reaction product of hydroxyethylcellulose with trimethylammonium-substituted epoxide, chloride, bromide and methosulfate salt of cetyltrimethylammonium and behenyltrimethylammonium, N-[3-(dimethylamino)propyl]octadecanamide, 1-octadecanol and 1-hexadecanol,
and mixtures thereof.

10. Use according to any one of Claims 1 to 9, **characterized in that** the cosmetically acceptable medium is water.

11. Use according to any one of Claims 1 to 10, **characterized in that** the cosmetic composition comprises further cosmetically customary additives, selected from the group of

surfactants, such as anionic, nonionic and amphoteric surfactants,
thickeners, gelling agents,
film formers,
moisturizing agents,
UV filters,
pearlizing agents,
vitamins,
antioxidants,
caffeine,
antidandruff active ingredients and preservatives.

12. Use according to any one of Claims 1 to 11, **characterized in that** the cosmetic composition is prepared by mixing at least one aqueous dispersion of precrosslinked organopolysiloxanes according to any one of Claims 1, 2 or 8 to 11 or prepared according to any one of Claims 3 to 7 with at least one conditioning agent and optionally further cosmetically customary additives in a cosmetically acceptable medium.


**Revendications**

1. Utilisation d'une composition cosmétique contenant, dans un milieu cosmétiquement acceptable, au moins un agent de conditionnement des cheveux, choisi dans le groupe formé par les polymères cationiques, les tensioactifs cationiques, les composés d'ammonium quaternaire ne se trouvant pas sous forme polymère, les organopolysiloxanes, les copolymères d'organopolysiloxane qui sont différents des organopolysiloxanes de formule (I) préréticulés, les esters d'acide gras et les alcools d'acide gras, les huiles et les cires naturelles ou synthétiques et le panthénol, les lipides, les protéines et les protéines hydrolysées ainsi que leurs mélanges, et au moins une dispersion aqueuse d'organopolysiloxanes préréticulés formés par des unités de formule générale

$$AKT_n R^2{}_b R_c (OR^1)_d SiO_{\frac{4-(n+b+c+d)}{2}} \qquad (I)$$

dans laquelle

R peut être identique ou différent et signifie un radical hydrocarboné monovalent, saturé ou insaturé, comprenant 1 à 200 atomes de carbone par radical, qui peut contenir un ou plusieurs hétéroatomes du groupe formé par N, P, S, O et halogène,
$R^1$ peut être identique ou différent et signifie un atome d'hydrogène ou un radical alkyle comprenant 1 à 8 atomes de carbone par radical,
$R^2$ peut être identique ou différent et signifie un radical hydrocarboné monovalent, le cas échéant substitué, comprenant 1 à 18 atomes de carbone par radical,
AKT représente un radical de formule $-CH_2NHR^4$, $-CH_2NR^4{}_2$ ou $-CH_2RN^5$

où

$R^4$ signifie un radical hydrocarboné monovalent comprenant 1 à 18 atomes de carbone, qui peut contenir un ou plusieurs hétéroatomes du groupe formé par N et O, et
$R^5$ signifie un radical hydrocarboné divalent comprenant 3 à 12 atomes de carbone, qui peut contenir un ou plusieurs hétéroatomes du groupe formé par N et O, et
b vaut 0, 1 ou 2,
c vaut 0, 1, 2 ou 3,
d vaut 0, 1 ou 2 et
n vaut 0, 1 ou 2,

sous réserve que la somme $n + b + c + d \le 3$ et qu'il existe en moyenne, par molécule, au moins un radical AKT, pour le traitement de fibres kératiniques, de préférence des cheveux.

2. Utilisation selon la revendication 1, **caractérisée en ce qu'**une dispersion aqueuse d'organopolysiloxanes préréticulés, constitués par des unités de formule générale

$$\mathrm{AKT}_n \mathrm{R^2}_b \mathrm{A}_m \mathrm{R'}_c (\mathrm{OR^1})_d \mathrm{SiO}_{\frac{4-(n+m+b+c+d)}{2}} \qquad (\mathrm{I'}) \ ,$$

est contenue comme dispersion aqueuse, AKT, $R^1$, $R^2$, b, c, d et n présentant la signification indiquée pour ceux-ci dans la revendication 1,

R' peut être identique ou différent et signifie un radical hydrocarboné monovalent comprenant 1 à 18 atomes de carbone,
A représente un radical amino de formule générale

$$-R^6-[NR^7-R^8-]_z NR^7_2,$$

dans laquelle

$R^6$ signifie un radical hydrocarboné divalent, linéaire ou ramifié, comprenant 3 à 18 atomes de carbone,
$R^7$ signifie un atome d'hydrogène, un radical alkyle comprenant 1 à 8 atomes de carbone ou un radical acyle,
$R^8$ signifie un radical hydrocarboné divalent comprenant 1 à 6 atomes de carbone,
z vaut 0, 1, 2, 3 ou 4 et
m vaut 0 ou 1,

sous réserve que la somme n + m + b + c + d ≤ 3 et que n et m dans la même unité siloxane ne valent pas simultanément 1 et qu'en moyenne, par molécule, au moins un radical AKT et un radical A sont contenus.

3. Utilisation selon la revendication 1 ou 2, **caractérisée en ce qu'**une dispersion aqueuse d'organopolysiloxanes préréticulés est préparée **en ce qu'**on transforme au moins un organopolysiloxane (1), constitué par des unités de formule générale

$$R_c (\mathrm{OR^1})_d \mathrm{SiO}_{\frac{4-(c+d)}{2}} \qquad (\mathrm{II})$$

dans laquelle R et $R^1$ ont la signification indiquée pour ceux-ci dans la revendication 1,

c vaut 0, 1, 2 ou 3 et
d vaut 0, 1 ou 2,

sous réserve que la somme c + d ≤ 3, et l'organopolysiloxane (1) contient en moyenne au moins un radical $OR^1$ par molécule, $R^1$ présentant de préférence la signification d'un atome d'hydrogène, avec au moins un silane (2) hautement réactif de formule générale

$$(\mathrm{AKT})_a R^2_b \mathrm{Si}(\mathrm{OR^3})_{4-(a+b)} \qquad (\mathrm{III})$$

ou ses hydrolysats partiels, dans laquelle AKT et $R^2$ présente la signification indiquée pour ceux-ci dans la revendication 1,

$R^3$ peut être identique ou différent et signifie un radical alkyle comprenant 1 à 8 atomes de carbone par radical,
a vaut 1 ou 2, de préférence 1, et
b vaut 0, 1 ou 2, de préférence 0 ou 1,

sous réserve que la somme a + b ≤ 3, en présence d'eau (3) et d'un émulsifiant (4),
sous réserve qu'on n'utilise pas conjointement de catalyseurs contenant un métal et que l'organopolysiloxane (1) et le silane (2) sont choisis de manière telle que les organopolysiloxanes sont préréticulés dans les dispersions obtenues.

4. Utilisation selon la revendication 3, **caractérisée en ce que** les organopolysiloxanes préréticulés ainsi préparés forment des films élastomères après l'élimination de l'eau.

**5.** Utilisation selon la revendication 3 ou 4, **caractérisée en ce qu'**on utilise, lors de la préparation de la dispersion aqueuse d'organopolysiloxanes préréticulés, comme organopolysiloxanes (1), ceux de formule générale

$$(R^1O)R'_2SiO(SiAR'O)_p(SiR'_2O)_qSiR'_2(OR^1) \qquad (IVc),$$

dans laquelle

R' peut être identique ou différent et signifie un radical hydrocarboné monovalent comprenant 1 à 18 atomes de carbone,
$R^1$ peut être identique ou différent et signifie un atome d'hydrogène ou un radical alkyle comprenant 1 à 8 atomes de carbone par radical,

le radical amino A présente la signification indiquée pour celui-ci dans la revendication 3,

p vaut un nombre de 1 à 1000, de préférence de 1 à 10, et
q vaut 0 ou un nombre entier de 1 à 2000, de préférence de 50 à 1000,

sous réserve que 10 à 100%, de préférence 20 à 100% de tous les radicaux $R^1$ sont des atomes d'hydrogène.

**6.** Utilisation selon la revendication 3, 4 ou 5, **caractérisée en ce que**, lors de la préparation de la dispersion aqueuse d'organopolysiloxanes préréticulés, on utilise, comme silane (2), au moins un silane trifonctionnel dans lequel a = 1 et b = 0.

**7.** Utilisation selon l'une quelconque des revendications 3 à 6, **caractérisée en ce que**, lors de la préparation de la dispersion aqueuse d'organopolysiloxanes préréticulés, le radical -$OR^3$ dans le silane (2) est un radical éthoxy.

**8.** Utilisation selon l'une quelconque des revendications 1 à 7, **caractérisée en ce que** le radical AKT est un radical cyclohexylaminométhyle ou un radical morpholinométhyle.

**9.** Utilisation selon l'une quelconque des revendications 1 à 8, **caractérisée en ce qu'**on utilise, comme agent de conditionnement des cheveux, un agent choisi dans le groupe constitué par le dérivé d'ammonium quaternaire d'une gomme de Cyamopsis tétragonoloba (Guar) modifiée par du propylèneglycoléther, le sel d'ammonium quaternaire polymère du produit de réaction de l'hydroxyéthylcellulose avec de l'époxyde substitué par du triméthylammonium, le sel de chlorure, de bromure et de méthosulfate du cétyltriméthylammonium et de béhényltriméthylammonium, le N-[3-(diméthylamino)propyl]octadécanamide, le 1-octadécanol et le 1-hexadécanol, ainsi que leurs mélanges.

**10.** Utilisation selon l'une quelconque des revendications 1 à 9, **caractérisée en ce que** le milieu cosmétiquement acceptable est l'eau.

**11.** Utilisation selon l'une quelconque des revendications 1 à 10, **caractérisée en ce que** la composition cosmétique contient d'autres additifs cosmétiquement usuels, choisis dans le groupe formé par les tensioactifs, tels que les tensioactifs anioniques, non ioniques et amphotères, les épaississants, les agents gélifiants, les agents filmogènes, les agents humectants, les filtres UV, les agents conférant un effet nacré, les vitamines, les antioxydants, la caféine, les agents antipelliculaires et les conservateurs.

**12.** Utilisation selon l'une quelconque des revendications 1 à 11, **caractérisée en ce que** la composition cosmétique est préparée par mélange d'au moins une dispersion aqueuse d'organopolysiloxanes préréticulés selon l'une quelconque des revendications 1, 2 ou 8 à 11 ou préparés selon l'une quelconque des revendications 3 à 7 avec au moins un agent de conditionnement et le cas échéant d'autres additifs cosmétiquement usuels dans un milieu cosmétiquement acceptable.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7223385 B2
- US 7485289 B2
- US 7220408 B2
- US 7504094 B2
- US 20080064813 A1

- EP 1921203 A1
- EP 992528 A2
- DE 19826081 A1
- DE 102010035844
- DE 102010020192

**In der Beschreibung aufgeführte Nicht-Patentliteratur**

- **M.D. BERTHIAUME.** Silicones in Hair Care. *Monograph,* 1997
- **J. SEJPKA.** Silicone in Haarpflegeprodukten. *SÖFW-Journal,* 1992, vol. 118 (17), 1065-1070
- Wetting Properties of Human Hair by Means of Dynamic Contact Angle Measurement. **R.A. LODGE ; B. BHUSAN.** Journal of Applied Polmyer Science. Wiley, 2006, vol. 102, 5255-5265
- The Chemistry and Manufacture of Cosmetics. **K. KRUMMEL ; STEPHANE CHIRON ; J. JACHOW-ICZ.** Formulating. 2000, vol. II, 359-396

- International Cosmetic Ingredient Dictionary & Handbook
- International Cosmetic Ingredient Dictionary & Handbook. The Personal Care Products Council. The Cosmetic, Toiletry, and Fragrance Association (CTFA), 2010
- Die Messung der Kämmkraft mit Doppelkammmethode nach Y.K. Kamath und Hans-Dietrich Weigmann. *J. Soc. Cosmet. Chem.,* 1986, vol. 37, 111-124